(19) Europäisches Patentamt
European Patent Office
Office européen des brevets

(11) EP 1 283 258 A1

(12) EUROPEAN PATENT APPLICATION

(43) Date of publication:
12.02.2003 Bulletin 2003/07

(51) Int Cl.[7]: C12N 9/00

(21) Application number: 02255559.3

(22) Date of filing: 08.08.2002

(84) Designated Contracting States:
AT BE BG CH CY CZ DE DK EE ES FI FR GB GR IE IT LI LU MC NL PT SE SK TR
Designated Extension States:
AL LT LV MK RO SI

(30) Priority: 09.08.2001 US 311063 P

(71) Applicant: Ortho-Clinical Diagnostics, Inc.
Rochester, NY 14626-5101 (US)

(72) Inventors:
- Heiskala, Marja
San Diego, California 92130 (US)
- Andersson, Leif Christer Leander
00200 Helsingfors (FI)
- Pitkanen, Laura
00320 Helsinki (FI)

(74) Representative: Fisher, Adrian John et al
CARPMAELS & RANSFORD
43 Bloomsbury Square
London WC1A 2RA (GB)

(54) **Ornithine decarboxylase similar protein and nucleic acid molecules encoding it**

(57) Purified and isolated Ornithine Decarboxylase-like protein (ODC-p) and variants of ODC-p have been prepared as have nucleic acids that encode for them. An assay for ODC-p is used to monitor disease progression and/or response to therapy.

EP 1 283 258 A1

## Description

### BACKGROUND

The invention relates to proteins related to Ornithine Decarboxylase ("ODC"), nucleic acids that encode for the ODC related proteins, and diagnostic and therapeutic uses of them.

The decarboxylation of ornithine to putrescine is the first step in the biosynthesis of the polyamines such as spermidine and spermine. The polyamines, which are found in animal tissues and microorganisms, are known to play an important role in cell growth and proliferation. ODC is the rate limiting enzyme in the biosynthesis of polyamines and is ubiquitously expressed in nucleated cells. Elevated catalytic activity of ODC is found in cancer and other rapidly proliferating cells. A low activity is typical of quiescent tissues with minimal cellular turnover.

ODC is subject to elaborate transcriptional regulation as well as post translational control in the form of a rapid physiologic turn over. The degradation of ODC occurs via binding to antizyme (AZ), which is a member of a protein family of three known homologues. The ODC-AZ complex is directed to proteasomal degradation without ubiquitination. The availability of AZ is regulated by an AZ-binding protein, antizyme inhibitor (AZI), which contains an AZ-binding site.

A human ODC-like protein, ODC-p, has now been isolated and purified. This protein is distributed in brain and gonadal tissue. Eight different, alternatively spliced cDNA variants have been cloned. Assays for the detection of ODC-p and its splice variants, particularly relative to the presence of ODC are useful in the detection and monitoring of diseases such as cancer and neurodegenerative disorders. They may also be used to monitor treatment of such diseases.

### SUMMARY OF THE INVENTION

In one aspect of the invention, purified and isolated ODC-p and variants of ODC-p have been prepared as have nucleic acids that encode for them. The biological and structural properties of these proteins are disclosed, as is the amino acid and nucleotide sequence. The recombinant DNA molecules, and portions thereof, are useful for isolating homologues of the DNA molecules, identifying and isolating genomic equivalents of the DNA molecules, and identifying, detecting or isolating mutant forms of the DNA molecules.

In another aspect of the invention, an assay for ODC-p and its splice variants is used to monitor disease progression and/or response to therapy.

### BRIEF DESCRIPTION OF THE DRAWING

Figure 1 - The nucleotide sequence of ODC-p is shown.
Figure 1a- The amino acid sequence of ODC-p is shown.
Figure 2 - The nucleotide sequence of SV2 is shown.
Figure 2a- The amino acid sequence of SV2 is shown.
Figure 3 - The nucleotide sequence of SV3 is shown.
Figure 3a- The amino acid sequence of SV3 is shown.
Figure 4 - The nucleotide sequence of SV4 is shown.
Figure 4a- The amino acid sequence of SV4 is shown.
Figure 5 - The nucleotide sequence of SV5 is shown.
Figure 5a- The amino acid sequence of SV5 is shown.
Figure 6 - The nucleotide sequence of SV6 is shown.
Figure 6a- The amino acid sequence of SV6 is shown.
Figure 7 - The nucleotide sequence of SV7 is shown.
Figure 7a- The amino acid sequence of SV7 is shown.
Figure 8 - The nucleotide sequence of SV8 is shown,
**[0022]** Figure 8a- The amino acid sequence of SV8 is shown.
Figure 9- The nucleotide sequences of exons I-11 are shown.

### DETAILED DESCRIPTION

ODC-P and its splice variants are proteins involved in cell differentiation. Vertebrate cells capable of producing ODC-P and its splice variants are found in the testes as well as in the central nervous system ("CNS").

The human genomic DNA sequence from the clone RPI-11703 (Gene Bank Accession AL020995) was used to define the gene structure. Sequence comparisons between ODC-p and its splice variants, ODC and AZI were performed using PlotSimilarity multiple alignment with blosum62 scoring matrix, and the ClustalW program. The homology between coding regions of ODC and ODC-p cDNAs is 59%. At the protein level, the homology between ODC and ODC-p is 54% and the similarity is 75%. The corresponding figures for AZI and ODC-p are 45% and 66%, respectively.

Comparing the coding regions of ODC and ODC-p and its splice variants, the homology is lowest in the 5'- and 3'-ends, but the central portion is conserved. This was shown, for example, through the coding region sequence (CDS) of human brain ODC-p that was sequenced with six primers covering each nucleotide of the whole coding region. It has also been found that the coding region (CDS) of ODC-p contains 11 exons while that of ODC consists of 10 exons. The exons of ODC and ODC-p (as well as AZI) are almost identical in size. However, the entire gene sequence of ODC-p spans 39.3 kb. The ODC gene sequence spans 7.9 kb.

Exon no. 9 of the ODC-p gene contains a premature stop-codon. This exon is not transcribed in the most abundant RT-PCR product from brain or testes although various clones produced from ESTs containing parts of ODC-p have been identified containing this exon.

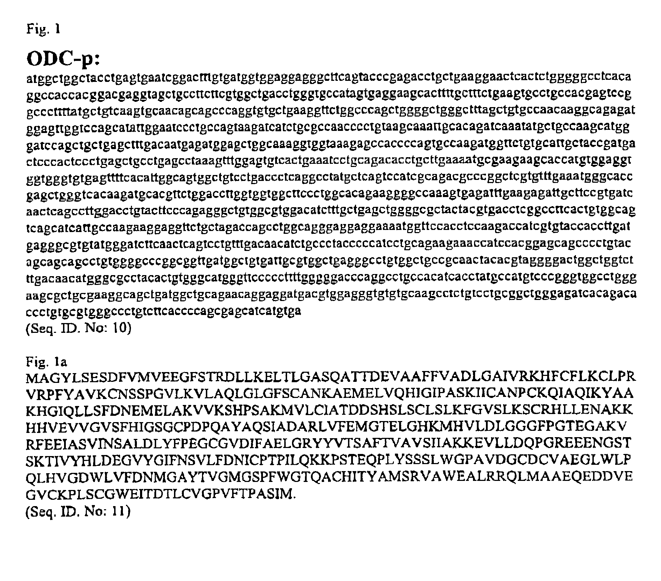

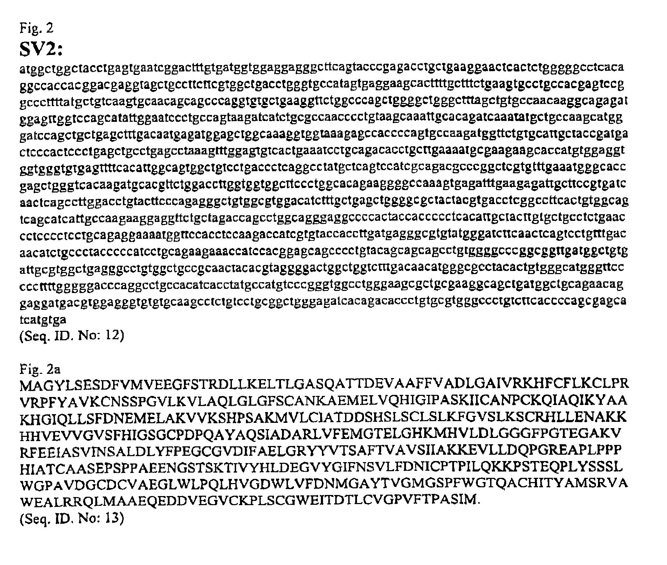

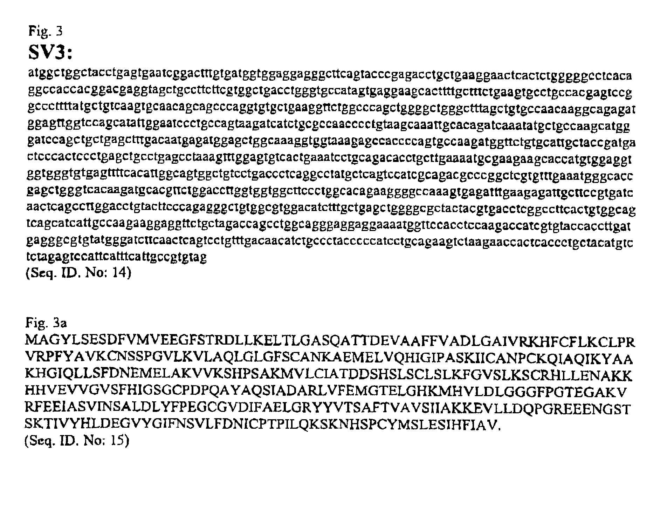

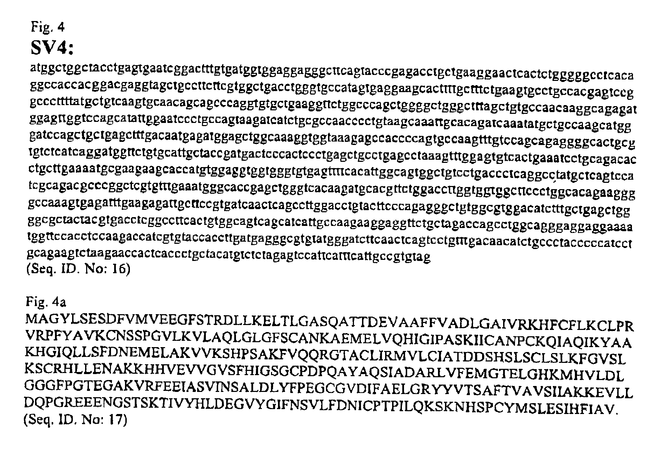

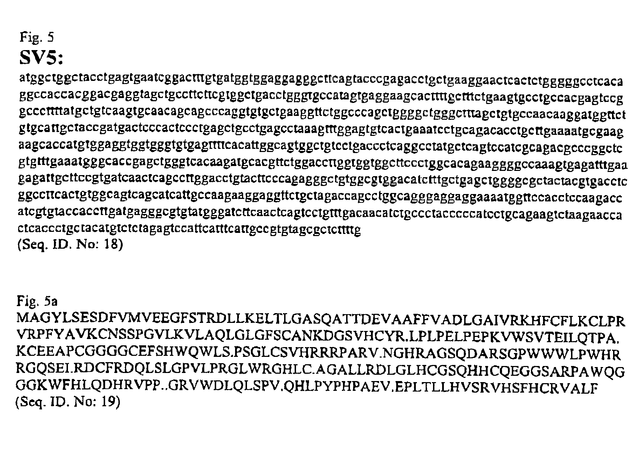

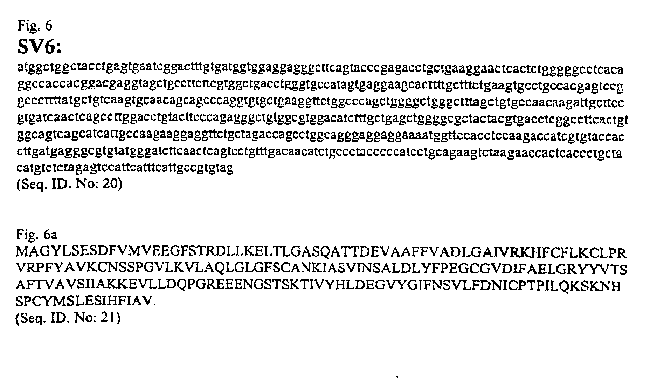

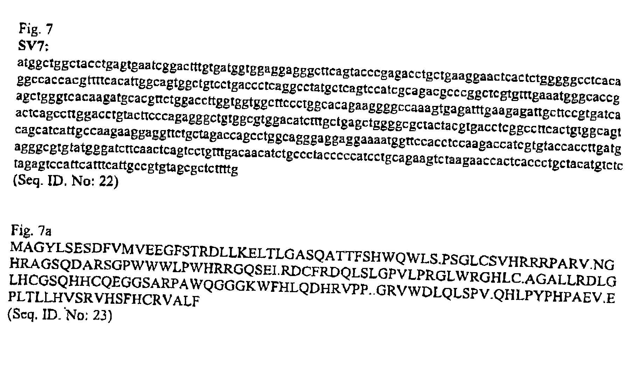

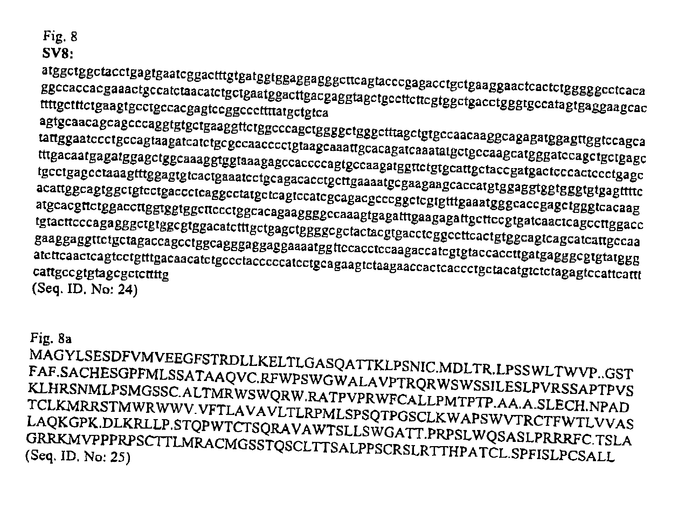

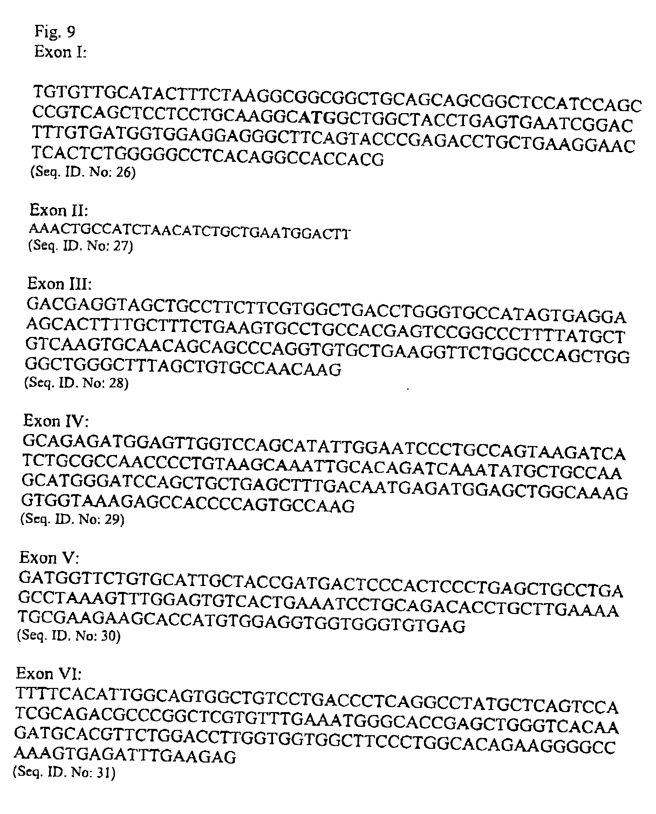

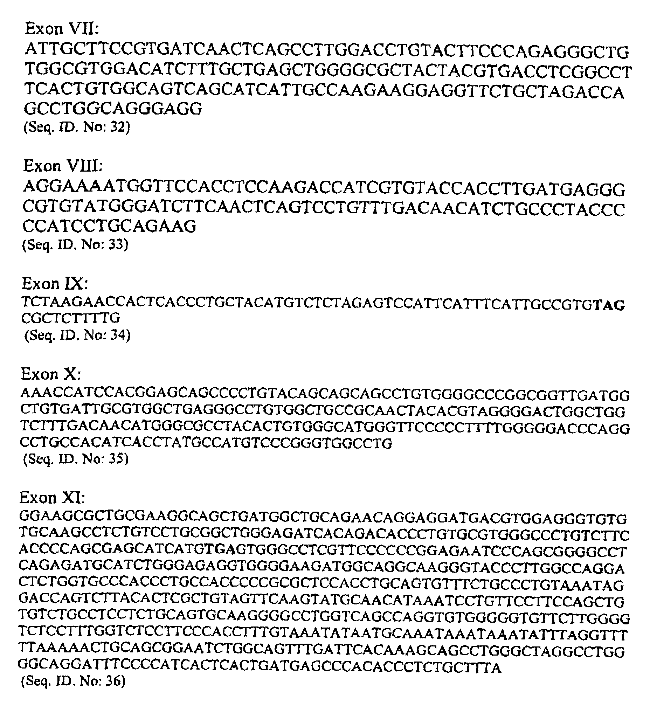

Six different alternatively spliced variants of ODC-p from testes and from adult brain were identified using primers specific to exon 9. By sequencing the clones another splicing variant terminating to exon 11 was also found. Thus, a total number of eight different, alternatively spliced isoforms of ODC-p with two different 3'-endings were obtained. ODC-p and SV2 are the longest variants of ODC-p. They are made up of the following exons: 1, 3-8, and 10-11, with and additional 60 base pair ("BP") addition to 5'-end of the exon no. 8 in the case of SV2. These isoforms are also the most easily copied from testis and adult brain. SV3 contains exons no. 1, 3-9; SV4 exons 1, 3-9 and an additive beginning in exon no. 5; SV5 exons 1, 3, 5—9; SV6 exons 1, 3, 7—9; SV7 exons 1, 6—9; and SV8 contains exons 1—9. Figures 1, 1a, 2, 2a, 3, 3a, 4, 4a, 5, 5a, 6, 6a, 7, 7a, 8, and 8a show the nucleic acid sequences that encode for ODC-p and its splice variants as well as the amino acid sequences of the corresponding proteins. Figure 9 shows the exons referred to above.

The stop codon is found at various locations in different isoforms of ODC-p. Shorter versions have their stop codon at exon 9 and have considerable variation in exons between start and stop. Exons 1, 7 and the 3'-end of exon 8 are consistently found in all isoforms. The shorter variants, however, do not include two sites for ODC-activity, C360 and D361. Different isoforms are expressed in different stages of ncuronal or spermatozoal differentiation. Indeed, greater amounts of shorter isoforms are found in the mRNA of the testis compared to that found in cells of the adult brain (where differentiation has already terminated).

There is a substantial amount of redundancy in the various codons that code for specific amino acids. Therefore, this invention is also directed to those nucleic acid sequences that contain alternative codons that code for the eventual translation of the claimed amino acids. Also included within the scope of this invention are mutations either in the DNA sequence or the translated protein, which do not substantially alter the ultimate physical properties of the expressed protein. For example, substitution of aliphatic amino acids alanine, valine, leucine and isoleucine; interchange of the hydroxyl residues serine and thrconine, exchange of the acidic residues aspartic acid and glutamic acid, substitution between the amide residues asparagine and glutamine, exchange of the basic residues lysine and arginine and ODC-p among the aromatic residues phenylalanine, tyrosine may not cause a change in functionality of the polypeptide. Such substitutions are well known and are described, for instance in Molecular Biology of the Gene, 4$^{th}$ Ed. Bengamin Cummings Pub. Co. by Watson *et al*.

DNA sequences coding for a peptide may be altered so as to code for a peptide having properties that are different than those of the naturally occurring peptide. Methods of altering the DNA sequences include, but are not limited to site directed mutagenesis, chimeric substitution, and gene fusions. Examples of altered properties include but are not limited to changes in the affinity of an enzyme for a substrate or a receptor for a ligand. All such changes of the polynucleotide or polypeptide sequences are anticipated as useful variants of the present invention.

Utilizing the BAC-clone sequence found from databanks, the chromosomal localization of the gene encoding ODC-p determined to be chromosome 1p33-34.1. The ODC gene is found on chromosome 2p25.

The clone RPI-1 1703 (Gene Bank Accession AL020995) was used to isolate human genomic ODC-p DNA. cDNAs for the alternatively spliced variants of ODC-p were amplified by RT-PCR using a 5' primer CAGCTCCTCCTGCAAGGCATGG (Seq. ID No.1) and a 3' primer GAGGCCCACTCACATGCTCGCT (Seq. ID No. 2), and human adult brain and testis tissue libraries as templates. An alternative 3' primer CTACACGGCAATGAAATGAATGGAC (Seq. ID No. 3) was also used for splice variants ending in the premature stop codon in exon IX. Further details are provided in Example I. Other cells and cell lines are also suitable for use to isolate ODC-p and its splice variants' mRNA. Selection of suitable cells can be done by screening for ODC-p and its splice variants' message in cell extracts or in whole cell assays. Cells that possess ODC-p message are suitable for the isolation of mRNA for ODC-p and its splice variants. This mRNA can then be reverse transcribed into a DNA strand, and the resulting cDNA can be amplified using PCR. Methods well-known in the art are readily used to molecularly clone nucleic acids encoding for ODC-p and its splice variants and prepare cDNA libraries. See, for example, Maniatis, T., Fritsch, E.F., Sambrook, J. Molecular Cloning: A Laboratory Manual, Second Edition, Cold Spring Harbor Laboratory Press, Cold Spring Harbor, NY. 1989.

Variant(s) of polynucleotides or polypeptides are polynucleotides or polypeptides that differ from a reference polynucleotide or polypeptide, respectively. A variant of the polynucleotide may be a naturally occurring variant such as a naturally occurring allelic variant, or it may be a variant that is not known to occur naturally. Such variants are within the scope of the invention and can include polynucleotide or polypeptide functional derivatives, degenerate variants, fragments, analogues, and homologues.

Polynucleotide(s) generally refers to any polyribonucleotide or polydeoxribonucleotide, which may be unmodified RNA or DNA or modified RNA or DNA. Thus, for instance, polynucleotides as used herein refers to, among others, single- and double-stranded DNA, DNA that is a mixture of single- and double-stranded regions or single-, double- and triple- stranded regions, single- and double-stranded RNA, and RNA that is mixture of single- and double-stranded regions, hybrid molecules comprising DNA and RNA that may be single-stranded or, more typically, double-stranded, or triple-stranded, or a mixture of single- and double-stranded regions. In addition, polynucleotide as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term polynucleotide includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. A great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including simple and complex cells, inter alia. Polynucleotides embraces short polynucleotides often referred to as oligonucleotide(s).

The polypeptides of the instant inventions include those referred to as peptides, oligopeptides, and oligomers (e.g., proteins) as well as their variants. Variants include, without limitation, those that have been subject to acetylation, acylation, ADP- ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, e.g., PROTEINS-- STRUCTURE AND MOLECULAR PROPERTIES, 2nd Ed., T. E. Creighton, W. H. Freeman and Company, New York (1993).

Determining identity and similarity are readily done according to well known methods such as those presented in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., (1988) SIAM J. Applied Math., 48, 1073. Methods to determine identity and similarity are codified in computer programs. Preferred computer program methods to determine identity and similarity between two sequences include, but are not limited to, GCG program package (Devereux, J., et al., (1984) Nucleic Acids Research 12(1), 387), BLASTP, BLASTN, and FASTA (Atschul, S. F. et al., (1990) J. Molec. Biol. 215, 403).

Preferred embodiments of the invention are polynucleotides that are at least 70% identical over their entire length to a polynucleotide encoding the polypeptide having the amino acid sequence set out in Seq. ID. No. 11, Seq. ID. No. 13, Seq. ID. No. 15, Seq. ID. No. 17, Seq. ID. No. 19, Seq. ID. No. 21, Seq. ID. No. 23, Seq. ID. No.25 and polynucleotides which are complementary to such polynucleotides. Alternatively, highly preferred are polynucleotides that comprise a region that is at least 80% identical, more highly preferred are polynucleotides at comprise a region that is at least 90% identical, and among these preferred polynucleotides, those with at least 95% are especially preferred. Furthermore, those with at least 97% identity are highly preferred among those with at least 95%, and among these those with at least 98% and at least 99% are particularly highly preferred, with at least 99% being the most preferred. The preferred polynucleotides which hybridize to the polynucleotides described above encode polypeptides which retain substantially the same biological function or activity as the polypeptide characterized by the deduced amino acid sequence of Seq. ID. No. 10, Seq. ID. No. 12, Seq. ID. No. 14, Seq. ID. No. 16, Seq. ID, No. 18, Seq. ID. No. 20, Seq. ID. No. 22, Seq. ID. No. 24_. Preferred embodiments in this respect, moreover, are polynucleotides that encode polypeptides that retain substantially the same biological function or activity as the mature polypeptide encoded by the DNA of Seq. ID. No. 10_, Seq. ID. No. 12. Seq. ID. No. 14, Seq. ID. No. 16, Seq. ID. No. 18, Seq. ID. No. 20, Seq. m. No. 22, Seq. ID. No. 24. The present invention further relates to polynucleotides that hybridize to the sequences described above, preferably to polynucleotides that hybridize under stringent conditions (those conditions in which hybridization will occur only if there is at least 95% and preferably at least 97% identity between the sequences).

Polynucleotides of the invention may be used as hybridization probes for mRNA, cDNA and genomic DNA to isolate full-length cDNAs and genomic clones of Seq. ID. No.11, Seq. ID. No. 13, Seq. ID. No. 15, Seq. ID. No. 17, Seq. ID. No.19, Seq. ID. No. 21, Seq. ID. No. 23, Seq. ID. No. 25 and to isolate cDNA and genomic clones of other genes that have a high sequence similarity. Such probes generally will comprise at least 15 bases. Preferably, such probes will have at least 30 bases and may have at least 50 bases. Particularly preferred probes will have at least 30 bases and will have 50 bases or less. For example, the coding region of the gene of the invention may be isolated by screening using the known DNA sequence to synthesize an oligonucleotide probe. A labeled oligonucleotide having a sequence complementary to that of a gene of the present invention is then used to screen a library of cDNA, genomic DNA or mRNA to determine to which members of the library the probe hybridizes.

The polypeptides of the present invention include the polypeptide Seq. ID. No. 11, Seq. ID. No. 13, Seq. ID. No. 15, Seq. ID. No. 17, Seq. ID. No. 19, Seq. ID. No. 21, Seq. ID. No. 23, Seq. ID. No. 25 (in particular the mature polypeptide) as well as polypeptides which have at least 70% identity to such polypeptides. Preferably, however, the polypeptides of the present invention have at least 80% identity, and more preferably at least 90% similarity (more preferably at least 90% identity) to the polypeptides. Still more preferably they have at least 95% similarity (still more preferably at least 97% identity) to the polypeptide of Seq. ID. No. 11, Seq. ID. No.13, Seq. ID. No.15, Seq. ID. No.17, Seq. ID. No. 19, Seq. ID. No. 21, Seq. ID. No. 23, Seq. ID. No. 25 and also include portions of such polypeptides with such portion of the polypeptide generally containing at least 30 amino acids and more preferably at least 50 amino acids.

The cloned DNA for ODC-p and its splice variants obtained through the methods described herein may be recombinantly expressed by molecular cloning into an expression vector containing a suitable promoter and other appropriate transcription regulatory elements, and transferred into prokaryotic or eukaryotic host cells to produce recombinant protein. Techniques for such manipulations are fully described in Maniatis, T, et al., supra.

A variety of mammalian, bacterial, and fungal expression vectors may be used to express recombinant ODC-p (and its splice variants) in mammalian cells. Commercially available mammalian expression vectors which may be suitable for recombinant ODC-p (and its splice variants) expression, include but are not limited to, pMAMneo (Clontech), pcDNA3 (Invitrogen), pMC1neo (Stratagene), pXT1 (Stratagene), pSG5 (Stratagene), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and lZD35 (ATCC 37565). pET vectors (Novagen) and pQE vectors (Qiagen) are preferred bacterial expression vectors. pYES2 (Invitrogen) and Pichia expression vector (Invitrogen) are preferred fungal vectors. pBlueBacII (Invitrogen) is a preferred insect cell expression vector. TOPO TA pCR 2.1 available from Invitrogen (Groeningen, the Netherlands) and pCIneo vector available from Promega (Madison, WI) are most preferred vectors.

Recombinant host cells may be prokaryotic or eukaryotic. Cell lines derived from mammalian species which may be suitable and which are commercially available, include but are not limited to, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, and HEK-293 (ATCC CRL1573),

The expression vector may be introduced into host cells via any one of a number of techniques including but not limited to transformation, transfection, protoplast fusion, lipofection, and electroporation. The expression vector-containing cells are clonally propagated and individually analyzed to determine whether they produce ODC-p protein and the splice variant proteins. Identification of ODC-p expressing host cell clones may be done by several means, including but not limited to immunological reactivity with anti-ODC-p antibodies, and the presence of host cell-associated ODC-p activity.

Expression of ODC-p DNA and that of the splice variants may also be performed using *in vitro* produced synthetic mRNA. Synthetic mRNA or mRNA isolated from ODC-p producing cells can be efficiently translated in various cell-free systems, including but not limited to wheat germ extracts and rcticulocyte extracts, as well as efficiently translated in cell based systems, including but not limited to microinjection into frog oocytes, with microinjection into frog oocytes being generally preferred.

Following expression, ODC-p and splice variant protein may be recovered to provide purified ODC-p and splice variant protein from cell lysates and extracts, or from conditioned culture medium, by various methods. These include, without limitation, combinations of, or individual application of salt fractionation, ion exchange chromatography, size exclusion chromatography, hydroxyapatite adsorption chromatography and hydrophobic interaction chromatography, lectin chromatography, and antibody/ligand chromatography.

Monospecific antibodies to ODC-p and its splice variants are purified from mammalian antisera containing antibodies reactive against ODC-p and its splice variants or are prepared as monoclonal antibodies reactive with ODC-p and its splice variants using the technique originally described by Kohler and Milstein, *Nature* 256: 495-497 (1975). Immunological techniques are described in, for example, Antibodies: A laboratory manual published by Cold Spring Harbor Laboratory Press, Cold Spring Harbor, NY, ISBN 0879693142. These methods may be utilized for producing monospecific antibodies specific for ODC-p polypeptide fragments (and those of its splice variants), or full-length nascent polypeptide, or the individual ODC-p subunits or those of its splice variants. Monospecific antibodies may be generated which are specific for only one ODC-p subunit or the fully functional ODC-p protein or those of its splice variants. Further, monospecific antibodies may be generated that inhibit normal function of ODC-p protein or that of its splice variants.

ODC-p antibody affinity columns are made by adding the antibodies to a gel support such that the antibodies form covalent linkages with the gel bead support. Preferred covalent linkages are made through amine, aldehyde, or sulfhydryl residues contained on the antibody.

Kits containing DNA or RNA of ODC-p and its splice variants, antibodies to ODC-p and its splice variants, or protien of ODC-p and its splice variants are also an aspect of the invention. Such kits are used to detect DNA which hybridizes to ODC-p (or its splice variants) DNA or to detect the presence of ODC-p protein or peptide fragments (or those of its splice variants) in a sample. Such characterization is useful for a variety of purposes including but not limited to disease diagnosis and monitoring, treatment monitoring, forensic analyses, diagnostic applications, and epidemiological studies.

The DNA molecules, RNA molecules, recombinant protein and antibodies of the present invention may be used to screen and measure levels of DNA, RNA or protein of ODC-p and its splice variants. The recombinant proteins, DNA molecules, RNA molecules and antibodies lend themselves to the formulation of kits suitable for the detection and typing of ODC-p. Such a kit comprises at least one container. Preferably, the containers are held in close proximity with a compartmentalized container and instructions. The kit further comprises reagents such as recombinant ODC-p protein or anti-ODC-p antibodies suitable for detecting ODC-p. Detection reagents such as labeled antigen or enzyme substrates or the like can also be incorporated in such a kit.

Nucleotide sequences that are complementary to the ODC-p encoding DNA sequence can be synthesized for antisense therapy. These antisense molecules may be DNA, stable derivatives of DNA such as phosphorothioates or methylphosphonates, RNA, stable derivatives of RNA such as 2'-*O*-alkylRNA, or other ODC-p antisense oligonucleotide mimetics. ODC-p antisense molecules may be introduced into cells by microinjection, liposome encapsulation or by expression from vectors harboring the antisense sequence. ODC-p antisense therapy may be particularly useful for the treatment of diseases where it is beneficial to reduce ODC-p activity. Protocols for molecular methodology of gene therapy suitable for use with the ODC-p gene is described in Gene Therapy Protocols, edited by Paul D. Robbins, Human press, Totawa NJ, 1996.

Pharmaceutically useful compositions comprising DNA, RNA, or protein, of ODC-p and its splice variants or modulators of ODC-p substrate binding activity or that of its splice variants, may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the protein, DNA, RNA, or modulator.

Therapeutic or diagnostic compositions of the invention are administered to an individual in amounts sufficient to treat or diagnose disorders in which modulation of ODC-p-related activity is indicated. The pharmaceutical compositions may be provided to the individual by a variety of routes such as subcutaneous, topical, oral and intramuscular.

In the methods of the present invention, the compounds or modulators herein described can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices. The compounds or modulators may alternatively be administered parenterally via injection of a formulation consisting of the active ingredient dissolved in an inert liquid carrier. Injection may be either intramuscular, intraluminal, intratracheal, or subcutaneous. The injectable formulation consists of the active ingredient mixed with an appropriate inert liquid carrier. Topical application of the compounds or modulators is possible through the use of a liquid drench or a shampoo containing the instant compounds or modulators as an aqueous solution or suspension.

## EXAMPLE 1

cDNAs for the alternatively spliced variants of ODC-p were amplified by PCR using a 5'-primer TCAGCTCCTCCTGCAAGGCATGG (SEQ. ID. NO. 1), designed according to the deduced ODC-p cDNA, beginning 10 nucleotides upstream from the predicted starting codon, and a 3'-primer GAGGCCCACTCACATGATGCTCGCT (SEQ. ID. NO. 4), ending nine nucleotides downstream from stop-codon in the exon XI, or an alternative 3'-primer CTACACGGCAATGAAATGAATGGAC (SEQ. ID. NO 3) ending in the premature stop-codon in exon IX, and using human adult brain and testis tissue-derived cDNA libraries as templates.

The PCR products were cloned into the TOPO TA pCR 2.1 vector (Invitrogen, Groeningen, the Netherlands), and sequenced using both vector and insert-derived primers by an automated sequencer (ABI 3100 Genetic Analyzer, Perkin-Elmer, Foster City, CA, USA).

**EXAMPLE 2 (Prophetic)**

Recombinantly produced ODC-p may be purified by antibody affinity chromatography.

ODC-p antibody affinity columns are made by adding the anti-ODC-p antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23 M glycine HCl (pH 2.6) to remove any non-conjugated antibody or extraneous protein. The column is then equilibrated in phosphate buffered saline (pH 7.3) together with appropriate membrane solubilizing agents such as detergents and the cell culture supernatants or cell extracts containing solubilized ODC-p are slowly passed through the column. The column is then washed with phosphate- buffered saline together with detergents until the optical density (A280) falls to background, then the protein is eluted with 0.23 M glycine-HCl (pH 2.6) together with detergents. The purified ODC-p protein is then dialyzed against phosphate buffered saline.

**EXAMPLE 3**

For the *In Vitro* translation, and COS-cell transfection, the cDNAs for ODC-p, its splicing variants and the cDNA for ODC were cloned into the pCIneo vector (Promega, Madison, WI, USA) at EcoRI site. The inserts were verified by sequencing.

ODC, ODC-p and its splice variants (1µg of pCIneo/insert) were translated by Promega's (Madison, WI, USA) TnT Coupled Reticulocyte Lysate System with T7 RNA polymerase according to manufacturers instructions. Redivue L-[$^{35}$S] Methionine (Amersham Pharmacia Biotech UK Limited) was used to label the proteins for visualization in Western blot. Translated, labeled proteins were separated on 8% SDS-PAGE and visualized by autoradiography.

The cDNA for ODC and ODC-p SVs 1, 2, 3, 5, 7 and 8, were translated in the system. Autoradiographic visualization showed approximately the same size, 51kDa, for ODC and SV2 of ODC-p, which is in concordance with the predicted protein sequences. The splicing variants 1, 3, 7 and 8 showed bands of 46 kd, 42 kd, 40 and 44kd, respectively, on SDS PAGE, corresponding to the protein sequences predicted from their cDNAs. The empty vector was used as a negative control.

**EXAMPLE 4**

The presence of the message for ODC-p and ODC in human tissues was determined by hybridizing human Multiple Tissue Expression (MTE) arrays (Clontech, Palo Alto, CA, USA) with specific probes. For detecting the message for ODC-p, a PCR product amplified using a 5' primer CACTCCCTGAGCTGC (Seq. ID. No. 5), and a 3' primer CTGCTCCGTGGATGGT (Seq. ID. No. 6) , selected from an ODC-p cDNA region with low homology to ODC, was cloned into pCR 2.1-TOPO vector. The insert (554 bp) was randomly labeled with Multiprime Labeling Kit (Amersham Pharmacia Biotech, UK). For detecting the message for ODC, the whole cDNA of ODC was randomly labeled. ODC-p probe was tested not to react with ODC or AZI, and the ODC probe was tested to be negative against ODC-p under the hybridization conditions used. Hybridization was performed following the manufacturers instructions (Clontech). The membrane was exposed both to x-ray films and phosphoimager plates and the intensities were analyzed using a MacBAS-program.

Dot blot revealed relatively high amounts of ODC-p mRNA in different parts of adult brain (cerebral cortex, cerebral lobes, cerebellum, basal nuclei, thalamus, substantia nigra, hippocampus, spinal cord), while a lower expression was seen in fetal brain. When hybridizing the same membrane with an ODC-specific probe, low signals were detected in the brain, with the exception of the pituitary gland while all the other human tissues showed a constant high expression. This reciprocal distribution of ODC-p and ODC in adult brain versus other human tissues illustrates the role that ODC-p has in replacing ODC in certain organs with low cellular turnover. No expression of ODC-p was seen in the rapidly growing, undifferentiated tumor cell lines, demonstrating that ODC-p is not demanded for proliferation, and emphasizing the distinctions of the functions of ODC-p and ODC.

A high expression of both ODC-p and ODC was seen in the testes. The ongoing spermatogenesis includes both rapid cell proliferation and terminal differentiation, which illustrates the need for both forms of the ODC-like proteins in this organ.

ODC is most useful to proliferating cells while ODC-p and its splice variants are most useful in cellular differentiation.

**EXAMPLE 5**

**Cell cultures and transient transfections:**

COS-7 cells were cultured in Dulbecco's modified Eagle's medium (DMEM) supplemented with 5% fetal calf serum (FCS), antibiotics and L-glutamine. COS-7 cells were transiently transfected to express ODC and the splice variants of ODC-p (pCIneo/insert), using Lipofectamine 2000 (Life Technologies Inc., Gibco BRL, CA). Twenty four hours after being transfected the cells were harvested, and the ODC activity of the cell lysates was measured.

**Determination of ODC-activity:**

Transfected cells were mechanically detached and collected in a lysis buffer containing 25 mM Tris-HCl (pH 7.4), 0.1 mM EDTA, 5 mM dithiothreitol and 0.1 mM pyridoxal-5-phosphate in which the cells were lysed by repeated freezing and thawing. The *in vitro* translation (ivt) products were diluted into the same buffer. The supernatant of the cell lysates and the diluted ivt-samples were added to a reaction buffer containing 0,2 M Tris-HCl, pH 7.7, 0,6 mM EDTA, 30 mM DTT, 0,4 mM PLP and [1-$^{14}$C]Ornithine hydrochloride (1,92 MBq/mmol; Amersham Pharmacia Biotech UK Limited, UK). After incubating for one hour at 37°C, the ODC activity was measured by determining the release of $^{14}CO_2$ from L-[1-$^{14}CO_2$] Ornithine.

As expected, the *in vitro* translated ODC was readily active. ODC-activity was not, however, reproducibly detected in any of the *in vitro* translation products of the cDNAs for ODC-p isoforms.

Transfectants with the ODC cDNA plasmid produced a high ODC activity measured by the conventional assay. Expression of the cDNA for the SV2 of ODC-p induced an ODC activity, above the background yet dramatically lower than that of ODC. The four other variants of ODC-p that were tested (SV1, 3, 4 and 8) did not increase omithine decarboxylase activity in transfected COS-7 cells.

**EXAMPLE 5 (Prophetic)**

**Enzyme Immunoassay Procedure**

Immunoassays are prepared for the ODC-p isoforms. This is achievable since detection of 10 fmol/L is possible in competitive assay. Sensitivity of noncompetitive assay is determined by lower limit of detection of the label used: 1 to 2,000,000 Zeptomoles ($10^{-21}$moles). Tietz Fundamentals of Clinical Chemistry" 4th Edition, p143.

Enzyme Immunoassay (EIA) procedure, antigen standards comprising a digest of normal adult brain tissue from epilepsia surgery (shown to contain the antigen by immunoperoxidase staining) are used. Human brain tissue specimens are pooled and homogenized in 10 volumes of 10 mM Tris buffer, pH 7.4, containing 0.2% (w/v) sodium deoxycholate at 4°C. The homogenate is quickly brought to 37 C and the following reagents (final concentration) are added while stirring: 1 mM cysteine (Sigma), 1 mM EDTA (Sigma), and papain (0.8 unit/ml) (Boehringer-Mannheim, Indianapolis, Ind.). After 5 minutes digestion is stopped by the addition of 5mM iodoacetamide (Sigma). The homogenate is centrifuged at 100,000 times/g for 1 hour at 4C, then extensively dialyzed against 10 mM Tris/0.9% NaCl solution buffer, pH 7.4, containing phenylmethysulfonyl fluoride and aminocaproic acid, each at 10 mM. The homogenate is frozen in small aliquots at a concentration of 0.5 mg of protein/mi.

The dose response curve that will be generated for the immunoassay procedure measuring ODC-p isoforms demonstrates linearity between antigen input of 100ng to 100µg/ml. For cerebrospinal liquor analysis, the range is 1ng to 1000ng/ml, since this is the range these samples are expected to contain the protein after dilution with the assay buffer.

Solid-phase preparations of polyclonal antibodies raised against the ODC-p splice variants 1 and 2 are prepared using CNBr-activated Sepharose (Pharmacia). Microtiter plates (Nunc I Immunoplates; Grand Island Biological Co., Grand Island, N.Y.) are coated with the antibodies (200 µl /well) in 50 mM carbonate-bicarbonate buffer, pH 9.6, for 18 hours at 4C. After removal of the antibody solution, residual protein binding sites on the plastic are blocked by the addition of 200 µl of assay buffer [PBS containing 1% (v/v) rabbit *serum* and 1% (w/v) bovine albumin]. After 1 hour of incubation at room temperature, the coated plates are used immediately for the assay procedure.

To perform the assay, 200 µl samples, diluted in assay buffer, are applied for 1-5 hours at 37C. After 3 washes using assay buffer, 200 µl of monoclonal anti-ODC-p antibody, covalently conjugated to horseradish peroxidase (Sigma, Type VI), is applied to each well for 1.5 hours at 37C. The conjugate is diluted to a concentration of 0.5 µg of immunoglobulin per ml of PBS containing 10% (v/v) murine serum. Following a wash procedure as above, 200 µl of substrate per well are applied for 0.5 hours at room temperature. Substrate solution contains 0.4 mg of o-phenylenediamine per ml of pH 5.0 citrate buffer and 0.003% hydrogen peroxide. The reaction is stopped by addition of 50 µl of 2N sulfuric acid, and absorbance is monitored at 488 nM using an enzyme assay plate reader (Fisher Scientific Co., Pittsburgh, Pa.).

The percentage of bound enzyme conjugate is calculated by the formula:

$$(B\text{-}B_0)(B_1 — B_0)(100)$$

where B=absorbance of the sample, $B_t$ =maximal absorbance, and $B_0$ =absorbance of the blank. Each assay is performed in triplicate using a standard digest and 26-fold diluted cerebrospinal fluid samples diluted in assay buffer. Specificity of the immunoassay is examined by substituting various antibody reagents at the solid phase, including an antibody to CEA and nonimmune rabbit serum. Of the solid phase antibodies only antibodies raised against ODC-p splice variants 1 and 2 bind antigen at high dilutions.

Levels of cerebrospinal fluid ODC-p splice variants 1 and 2 are detected for normal control subjects, patients with benign and malignant brain tumors, patients with epilepsia, patients with multiple myeloma, patients with anoxic brain trauma, patients with multi-infarction dementia, and patients with neurodegenerative diseases, such as Alzheimer's disease and Parkinson's disease.

Cerebrospinal fluid obtained from apparently healthy individuals exhibits a mean value of approximately 90ng/ml of ODC-p/ml. Only 5% of the samples express cerebrospinal fluid antigen at 300ng/ml or above, and this value is chosen as cutoff for elevated cerebrospinal fluid levels.

Cerebrospinal fluid from patients with benign or malignant brain tumors exhibit a mean value of 160ng/ml, with a large range of variation, depending on the amount of cell death associated with the tumor.

Cerebrospinal fluid obtained from multiple myeloma exhibits close to normal levels of ODC-p in the cerebrospinal fluid.

Cerebrospinal fluid from patients with anoxic brain trauma exhibits the mean value of 500ng/ml of ODC-p at the acute phase, with a rapid decline to normal levels.

Cerebrospinal fluid from patients with clinical multi-infarction dementia exhibits a mean value of 300ng/ml of ODC-p.

Cerebrospinal fluid from patients with neurodegenerative diseases exhibits a mean value of 600ng/ml of ODC-p.

The assay is conducted at regular intervals over the course of treatment of a patient. After treatment, a significant decrease in cerebrospinal fluid ODC-p occurs. Using a limited number of patients who, after the diagnosis, have been treated with regimens that significantly improve their clinical performance, a quantitative relationship between cerebrospinal fluid ODC-p and the amount of cell death in the central nervous system is established. Such measurements thereby facilitate patient monitoring and can also assist in diagnosing, and predicting the clinical outcome, of dementia patients.

## EXAMPLE 6 (Prophetic)

### Quantitative RT-PCR Based Screening of ODC-p and ODC Expression Levels in Ejaculate

The technique consists of three parts: extraction of total RNA from the sample, synthesis of cDNA from RNA by reverse transcription (RT), and amplification of a specific cDNA by polymerase chain reaction (PCR).

RNA is first extracted from an ejaculate specimen, centrifuged at 1500 rpm for 15 minutes to harvest the cells shed in the fluid. Using Ambion RNAWiz (cat 9736), total RNA is extracted with a yield of 100ng of RNA per 100µg of specimen. The sample is homogenized at 1ml of RNAWiz, incubated for 5 minutes at room temperature (RT), and mixed with 200µl of chloroform. The mixture is shaken for 20 seconds, incubated at RT for ten minutes, and centrifuged at +4°C at 12,000 rpm for 15 minutes. The upper phase, with the RNA, is then transferred into another Eppendorf tube, and mixed with 500µof Rnase free water. After mixing 1ml of isopropanol is added, and the solution is incubated at RT for 10 minutes. Thereafter the mixture is centrifuged at 12,000 rpm for 15 minutes, the supernatant is removed with a pipet, and 1ml of 75% ethanol is added. After mixing the sample is centrifuged at +4°C (12,000 rpm) for 10 minutes, the supernatant is removed, and the pellet is air dried. The RNA is then dissolved in water, the concentration is measured, and an aliquot is used for the RT reaction.

Using Ambion's RETROscript™ Kit, the one-hour, 42°C RT reaction is mediated by a retroviral reverse transcriptase.

An aliquot of the resultant cDNA is exponentially amplified via cycles of denaturation, annealing and extension (PCR) using specific primers designed for ODC, and ODC-p splice variants 1 and 2. The primers are selected at intron spanning areas to eliminate possible DNA contamination. Primers are designed to cover those areas that are the most dissimilar in each of the molecules, in order to optimize specificity. ODC-p is selectively amplified by primers 5'CTTTTGCTTTCTGAAGTG (Seq. ID. No.7) and 3'CTGCTCCGTGGATGGT (Seq. ID. No. 6) and ODC by primers 5'AAAGCAGTCTGTCGTCTC (Seq. ID. No. 8) and 3'GCGCTCAACAATCCGAT (Seq. ID. No. 9).

Relative quantitation is used to compare different samples' transcript abundance across multiple samples, using a co-amplified internal control, 18S rRNA, for sample normalization. Results are expressed as ratios of the gene specific signal to the internal control signal. This yields a corrected relative value for the gene specific product in each sample. If necessary, a synthetic RNA competitor transcript is synthesized and used to identify compatible linear range conditions where the PCR products of the molecules of interest are detectable.

Samples of ejaculates are collected from obviously healthy individuals, patients with prostate cancer, patients with testicular cancer, patients with benign prostate hyperplasia, patients with prostatitis, patients with pyelonephritis, patients with urethritis, and patients with oligozoospermia and azoospermia. The ratio of the expression of ODC versus ODC-p is from 0.7 to 1.3 in the samples of healthy individuals, with values exceeding 20 or being lower than 0.4 indicating a disease state. In samples of the patients with malignant proliferative diseases in the testes the mean value of the ratio of ODC to ODC-p is 100. In samples of the patients with inflammatory diseases the mean value of the ratio is 30. In samples of the patients with defective spermatogenesis the mean value of the ratio of ODC to ODC-p is 0.02. In the samples of patients with diseases in the other organs in the genitourinary tract the ratio of the expression of ODC to ODC-p is in the normal range, except for patients with prostate cancer, where the ratio is between 20 and 100, depending on the stage. Successful treatment of both malignant and inflammatory diseases of the testes, and carcinoma of the prostate, normalizes the ratio quickly. Measuring the ratio of the expression of ODC versus ODC-p in the ejaculates helps to diagnose correctly individuals with fertility problems, and screens for early testicular cancer. The measurement can also be made from testicular biopsy.

## Claims

1. An isolated and purified molecule selected from a group consisting of:

   (a) a nucleic acid molecule encoding a protein having at least a 70% identity to a polypeptide selected from the group consisting of SEQ ID NO. 13, SEQ ID NO. 15. SEQ ID NO. 17, SEQ ID NO. 19, SEQ ID NO. 21, SEQ ID NO. 23, and SEQ ID NO. 25;
   (b) a nucleic acid molecule which is complementary to the nucleic acid of (a);
   (c) a nucleic acid molecule comprising at least 15 sequential bases of the nucleic acid of (a) or (b); and
   (d) a nucleic acid molecule that hybridizes under stringent conditions to the nucleic acid molecule of (a)

2. The isolated and purified nucleic acid molecule of claim 1, having a nucleotide sequence selected from a group consisting of: SEQ ID.NO. 10, SEQ ID NO. 12, SEQ ID NO. 14, SEQ ID NO.16, SEQ ID NO. 18, SEQ ID NO. 20, SEQ ID NO. 22, and SEQ ID NO. 24.

3. A method of assessing the health of a patient comprising: detecting the expression level of the nucleic acid encoding for ODC-p or a splice variant and correlating the presence or quantity of such molecule with the presence or absence of dysfunction.

4. The method of claim 3 used to diagnose CNS function, monitor CNS disease, or monitor the treatment of CNS disease.

5. The method of claim 3 used for the purpose of diagnosing testicular function, monitoring testicular dysfunction, or monitoring the treatment of testicular dysfunction.

6. A method of assessing the health of a patient comprising: detecting the amount of ODC-p or a splice variant thereof in a patient sample and correlating the presence or quantity of ODC with the presence or abscnce of dysfunction.

7. The method of claim 6 used for the purpose of diagnosing testicular function, monitoring testicular dysfunction, or monitoring the treatment of testicular dysfunction.

8. The method of claim 6 used to diagnose CNS function, monitor CNS disease, or monitor the treatment of CNS disease

9. A kit for determining the presence of ODC-p or a splice variant thereof comprising:

    a) antibody immunologically reactive with ODC-p or a splice variant thereof, and
    b) reagents for detecting the presence of the antibody.

10. A kit for determining the presence of a first nucleic acid molecule which encodes ODC-p, splice variants thereof, or a first nucleic acid molecule encoding a protein having at least a 70% identity to a polypeptide comprising amino acids of SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, and SEQ ID NO. 25, said kit comprising:

    a) a second nucleic acid molecule that is complementary a portion of the nucleic acid sequences that encode ODC-p wherein said second nucleic acid molecule comprises at least 15 sequential bases of the first nucleic acid sequence; and
    b) reagents for detecting the presence of the second nucleic acid molecule.

Fig. 1

## ODC-p:

atggctggctacctgagtgaatcggactttgtgatggtggaggagggcttcagtacccgagacctgctgaaggaactcactctgggggcctcaca
ggccaccacggacgaggtagctgccttcttcgtggctgacctgggtgccatagtgaggaagcacttttgctttctgaagtgcctgccacgagtccg
gcccttttatgctgtcaagtgcaacagcagcccaggtgtgctgaaggttctggcccagctggggctgggctttagctgtgccaacaaggcagagat
ggagttggtccagcatattggaatccctgccagtaagatcatctgcgccaacccctgtaagcaaattgcacagatcaaatatgctgccaagcatgg
gatccagctgctgagctttgacaatgagatggagctggcaaaggtggtaaagagccaccccagtgccaagatggttctgtgcattgctaccgatga
ctcccactccctgagctgcctgagcctaaagtttggagtgtcactgaaatcctgcagacacctgcttgaaaatgcgaagaagcaccatgtggaggt
ggtgggtgtgagttttcacattggcagtggctgtcctgaccctcaggcctatgctcagtccatcgcagacgcccggctcgtgtttgaaatgggcacc
gagctgggtcacaagatgcacgttctggaccttggtggtggcttccctggcacagaaggggccaaagtgagatttgaagagattgcttccgtgatc
aactcagccttggacctgtacttcccagagggctgtggcgtggacatctttgctgagctggggcgctactacgtgacctcggccttcactgtggcag
tcagcatcattgccaagaaggaggttctgctagaccagcctggcagggaggaggaaaatggttccacctccaagaccatcgtgtaccaccttgat
gagggcgtgtatgggatcttcaactcagtcctgtttgacaacatctgccctacccccatcctgcagaagaaaccatccacggagcagcccctgtac
agcagcagcctgtggggcccggcggttgatggctgtgattgcgtggctgagggcctgtggctgccgcaactacacgtaggggactggctggtct
ttgacaacatgggcgcctacactgtgggcatgggttcccccttttgggggacccaggcctgccacatcacctatgccatgtcccgggtggcctggg
aagcgctgcgaaggcagctgatggctgcagaacaggaggatgacgtggagggtgtgtgcaagcctctgtcctgcggctgggagatcacagaca
ccctgtgcgtgggccctgtcttcaccccagcgagcatcatgtga
(Seq. ID. No: 10)

Fig. 1a
MAGYLSESDFVMVEEGFSTRDLLKELTLGASQATTDEVAAFFVADLGAIVRKHFCFLKCLPR
VRPFYAVKCNSSPGVLKVLAQLGLGFSCANKAEMELVQHIGIPASKIICANPCKQIAQIKYAA
KHGIQLLSFDNEMELAKVVKSHPSAKMVLCIATDDSHSLSCLSLKFGVSLKSCRHLLENAKK
HHVEVVGVSFHIGSGCPDPQAYAQSIADARLVFEMGTELGHKMHVLDLGGGFPGTEGAKV
RFEEIASVINSALDLYFPEGCGVDIFAELGRYYVTSAFTVAVSIIAKKEVLLDQPGREEENGST
SKTIVYHLDEGVYGIFNSVLFDNICPTPILQKKPSTEQPLYSSSLWGPAVDGCDCVAEGLWLP
QLHVGDWLVFDNMGAYTVGMGSPFWGTQACHITYAMSRVAWEALRRQLMAAEQEDDVE
GVCKPLSCGWEITDTLCVGPVFTPASIM.
(Seq. ID. No: 11)

Fig. 2

**SV2:**

atggctggctacctgagtgaatcggactttgtgatggtggaggagggcttcagtacccgagacctgctgaaggaactcactctgggggcctcaca
ggccaccacggacgaggtagctgccttcttcgtggctgacctgggtgccatagtgaggaagcacttttgctttctgaagtgcctgccacgagtccg
gcccttttatgctgtcaagtgcaacagcagcccaggtgtgctgaaggttctggcccagctggggctgggctttagctgtgccaacaaggcagagat
ggagttggtccagcatattggaatccctgccagtaagatcatctgcgccaacccctgtaagcaaattgcacagatcaaatatgctgccaagcatgg
gatccagctgctgagctttgacaatgagatggagctggcaaaggtggtaaagagccaccccagtgccaagatggttctgtgcattgctaccgatga
ctcccactcccctgagctgcctgagcctaaagtttggagtgtcactgaaatcctgcagacacctgcttgaaaatgcgaagaagcaccatgtggaggt
ggtgggtgtgagttttcacattggcagtggctgtcctgaccctcaggcctatgctcagtccatcgcagacgcccggctcgtgtttgaaatgggcacc
gagctgggtcacaagatgcacgttctggaccttggtggtggcttccctggcacagaaggggccaaagtgagatttgaagagattgcttccgtgatc
aactcagccttggacctgtacttcccagagggctgtggcgtggacatctttgctgagctggggcgctactacgtgacctcggccttcactgtggcag
tcagcatcattgccaagaaggaggttctgctagaccagcctggcagggaggccccactaccacccccctcacattgctacttgtgctgcctctgaac
cctcccctcctgcagaggaaaatggttccacctccaagaccatcgtgtaccaccttgatgagggcgtgtatgggatcttcaactcagtcctgtttgac
aacatctgccctacccccatcctgcagaagaaaccatccacggagcagcccctgtacagcagcagcctgtggggcccggcggttgatggctgtg
attgcgtggctgagggcctgtggctgccgcaactacacgtaggggactggctggtctttgacaacatgggcgcctacactgtgggcatgggttcc
cccttttggggggacccaggcctgccacatcacctatgccatgtcccgggtggcctgggaagcgctgcgaaggcagctgatggctgcagaacag
gaggatgacgtggagggtgtgtgcaagcctctgtcctgcggctgggagatcacagacaccctgtgcgtgggccctgtcttcaccccagcgagca
tcatgtga

(Seq. ID. No: 12)

Fig. 2a

MAGYLSESDFVMVEEGFSTRDLLKELTLGASQATTDEVAAFFVADLGAIVRKHFCFLKCLPR
VRPFYAVKCNSSPGVLKVLAQLGLGFSCANKAEMELVQHIGIPASKIICANPCKQIAQIKYAA
KHGIQLLSFDNEMELAKVVKSHPSAKMVLCIATDDSHSLSCLSLKFGVSLKSCRHLLENAKK
HHVEVVGVSFHIGSGCPDPQAYAQSIADARLVFEMGTELGHKMHVLDLGGGFPGTEGAKV
RFEEIASVINSALDLYFPEGCGVDIFAELGRYYVTSAFTVAVSIIAKKEVLLDQPGREAPLPPP
HIATCAASEPSPPAEENGSTSKTIVYHLDEGVYGIFNSVLFDNICPTPILQKKPSTEQPLYSSSL
WGPAVDGCDCVAEGLWLPQLHVGDWLVFDNMGAYTVGMGSPFWGTQACHITYAMSRVA
WEALRRQLMAAEQEDDVEGVCKPLSCGWEITDTLCVGPVFTPASIM.

(Seq. ID. No: 13)

Fig. 3

**SV3:**

atggctggctacctgagtgaatcggactttgtgatggtggaggagggcttcagtacccgagacctgctgaaggaactcactctgggggcctcaca
ggccaccacggacgaggtagctgccttcttcgtggctgacctgggtgccatagtgaggaagcacttttgctttctgaagtgcctgccacgagtccg
gcccttttatgctgtcaagtgcaacagcagcccaggtgtgctgaaggttctggcccagctggggctgggctttagctgtgccaacaaggcagagat
ggagttggtccagcatattggaatccctgccagtaagatcatctgcgccaacccctgtaagcaaattgcacagatcaaatatgctgccaagcatgg
gatccagctgctgagctttgacaatgagatggagctggcaaaggtggtaaagagccaccccagtgccaagatggttctgtgcattgctaccgatga
ctcccactccctgagctgcctgagcctaaagtttggagtgtcactgaaatcctgcagacacctgcttgaaaatgcgaagaagcaccatgtggaggt
ggtgggtgtgagttttcacattggcagtggctgtcctgaccctcaggcctatgctcagtccatcgcagacgcccggctcgtgtttgaaatgggcacc
gagctgggtcacaagatgcacgttctggaccttggtggtggcttccctggcacagaaggggccaaagtgagatttgaagagattgcttccgtgatc
aactcagccttggacctgtacttcccagagggctgtggcgtggacatctttgctgagctggggcgctactacgtgacctcggccttcactgtggcag
tcagcatcattgccaagaaggaggttctgctagaccagcctggcagggaggaggaaaatggttccacctccaagaccatcgtgtaccaccttgat
gagggcgtgtatgggatcttcaactcagtcctgtttgacaacatctgccctacccccatcctgcagaagtctaagaaccactcaccctgctacatgtc
tctagagtccattcatttcattgccgtgtag
(Seq. ID. No: 14)

Fig. 3a

MAGYLSESDFVMVEEGFSTRDLLKELTLGASQATTDEVAAFFVADLGAIVRKHFCFLKCLPR
VRPFYAVKCNSSPGVLKVLAQLGLGFSCANKAEMELVQHIGIPASKIICANPCKQIAQIKYAA
KHGIQLLSFDNEMELAKVVKSHPSAKMVLCIATDDSHSLSCLSLKFGVSLKSCRHLLENAKK
HHVEVVGVSFHIGSGCPDPQAYAQSIADARLVFEMGTELGHKMHVLDLGGGFPGTEGAKV
RFEEIASVINSALDLYFPEGCGVDIFAELGRYYVTSAFTVAVSIIAKKEVLLDQPGREEENGST
SKTIVYHLDEGVYGIFNSVLFDNICPTPILQKSKNHSPCYMSLESIHFIAV.
(Seq. ID. No: 15)

Fig. 4

**SV4:**

atggctggctacctgagtgaatcggactttgtgatggtggaggagggcttcagtacccgagacctgctgaaggaactcactctgggggcctcaca
ggccaccacggacgaggtagctgccttcttcgtggctgacctgggtgccatagtgaggaagcacttttgctttctgaagtgcctgccacgagtccg
gcccttttatgctgtcaagtgcaacagcagcccaggtgtgctgaaggttctggcccagctggggctgggctttagctgtgccaacaaggcagagat
ggagttggtccagcatattggaatccctgccagtaagatcatctgcgccaacccctgtaagcaaattgcacagatcaaatatgctgccaagcatgg
gatccagctgctgagctttgacaatgagatggagctggcaaaggtggtaaagagccaccccagtgccaagtttgtccagcagaggggcactgcg
tgtctcatcaggatggttctgtgcattgctaccgatgactcccactccctgagctgcctgagcctaaagtttggagtgtcactgaaatcctgcagacac
ctgcttgaaaatgcgaagaagcaccatgtggaggtggtgggtgtgagtttcacattggcagtggctgtcctgaccctcaggcctatgctcagtcca
tcgcagacgcccggctcgtgtttgaaatgggcaccgagctgggtcacaagatgcacgttctggaccttggtggtggcttccctggcacagaaggg
gccaaagtgagatttgaagagattgcttccgtgatcaactcagccttggacctgtacttcccagagggctgtggcgtggacatctttgctgagctgg
ggcgctactacgtgacctcggccttcactgtggcagtcagcatcattgccaagaaggaggttctgctagaccagcctggcagggaggaggaaaa
tggttccacctccaagaccatcgtgtaccaccttgatgagggcgtgtatgggatcttcaactcagtcctgtttgacaacatctgccctacccccatcct
gcagaagtctaagaaccactcaccctgctacatgtctctagagtccattcatttcattgccgtgtag
(Seq. ID. No: 16)

Fig. 4a

MAGYLSESDFVMVEEGFSTRDLLKELTLGASQATTDEVAAFFVADLGAIVRKHFCFLKCLPR
VRPFYAVKCNSSPGVLKVLAQLGLGFSCANKAEMELVQHIGIPASKIICANPCKQIAQIKYAA
KHGIQLLSFDNEMELAKVVKSHPSAKFVQQRGTACLIRMVLCIATDDSHSLSCLSLKFGVSL
KSCRHLLENAKKHHVEVVGVSFHIGSGCPDPQAYAQSIADARLVFEMGTELGHKMHVLDL
GGGFPGTEGAKVRFEEIASVINSALDLYFPEGCGVDIFAELGRYYVTSAFTVAVSIIAKKEVLL
DQPGREEENGSTSKTIVYHLDEGVYGIFNSVLFDNICPTPILQKSKNHSPCYMSLESIHFIAV.
(Seq. ID. No: 17)

Fig. 5

**SV5:**

atggctggctacctgagtgaatcggactttgtgatggtggaggagggcttcagtacccgagacctgctgaaggaactcactctgggggcctcaca
ggccaccacggacgaggtagctgccttcttcgtggctgacctgggtgccatagtgaggaagcacttttgctttctgaagtgcctgccacgagtccg
gcccttttatgctgtcaagtgcaacagcagcccaggtgtgctgaaggttctggcccagctggggctgggctttagctgtgccaacaaggatggttct
gtgcattgctaccgatgactcccactccctgagctgcctgagcctaaagtttggagtgtcactgaaatcctgcagacacctgcttgaaaatgcgaag
aagcaccatgtggaggtggtgggtgtgagttttcacattggcagtggctgtcctgaccctcaggcctatgctcagtccatcgcagacgcccggctc
gtgtttgaaatgggcaccgagctgggtcacaagatgcacgttctggaccttggtggtggcttccctggcacagaaggggccaaagtgagatttgaa
gagattgcttccgtgatcaactcagccttggacctgtacttcccagagggctgtggcgtggacatctttgctgagctggggcgctactacgtgacctc
ggccttcactgtggcagtcagcatcattgccaagaaggaggttctgctagaccagcctggcagggaggaggaaaatggttccacctccaagacc
atcgtgtaccaccttgatgagggcgtgtatgggatcttcaactcagtcctgtttgacaacatctgccctacccccatcctgcagaagtctaagaacca
ctcaccctgctacatgtctctagagtccattcatttcattgccgtgtagcgctcttttg
(Seq. ID. No: 18)

Fig. 5a

MAGYLSESDFVMVEEGFSTRDLLKELTLGASQATTDEVAAFFVADLGAIVRKHFCFLKCLPR
VRPFYAVKCNSSPGVLKVLAQLGLGFSCANKDGSVHCYR.LPLPELPEPKVWSVTEILQTPA.
KCEEAPCGGGGCEFSHWQWLS.PSGLCSVHRRRPARV.NGHRAGSQDARSGPWWWLPWHR
RGQSEI.RDCFRDQLSLGPVLPRGLWRGHLC.AGALLRDLGLHCGSQHHCQEGGSARPAWQG
GGKWFHLQDHRVPP..GRVWDLQLSPV.QHLPYPHPAEV.EPLTLLHVSRVHSFHCRVALF
(Seq. ID. No: 19)

Fig. 6

**SV6:**

atggctggctacctgagtgaatcggactttgtgatggtggaggagggcttcagtacccgagacctgctgaaggaactcactctggggggcctcaca
ggccaccacggacgaggtagctgccttcttcgtggctgacctgggtgccatagtgaggaagcacttttgctttctgaagtgcctgccacgagtccg
gcccttttatgctgtcaagtgcaacagcagcccaggtgtgctgaaggttctggcccagctggggctgggctttagctgtgccaacaagattgcttcc
gtgatcaactcagccttggacctgtacttcccagagggctgtggcgtggacatctttgctgagctggggcgctactacgtgacctcggccttcactgt
ggcagtcagcatcattgccaagaaggaggttctgctagaccagcctggcagggaggaggaaaatggttccacctccaagaccatcgtgtaccac
cttgatgagggcgtgtatgggatcttcaactcagtcctgtttgacaacatctgccctacccccatcctgcagaagtctaagaaccactcaccctgcta
catgtctctagagtccattcatttcattgccgtgtag
(Seq. ID. No: 20)

Fig. 6a

MAGYLSESDFVMVEEGFSTRDLLKELTLGASQATTDEVAAFFVADLGAIVRKHFCFLKCLPR
VRPFYAVKCNSSPGVLKVLAQLGLGFSCANKIASVINSALDLYFPEGCGVDIFAELGRYYVTS
AFTVAVSIIAKKEVLLDQPGREEENGSTSKTIVYHLDEGVYGIFNSVLFDNICPTPILQKSKNH
SPCYMSLESIHFIAV.
(Seq. ID. No: 21)

Fig. 7
**SV7:**
atggctggctacctgagtgaatcggactttgtgatggtggaggagggcttcagtacccgagacctgctgaaggaactcactctgggggcctcaca
ggccaccacgttttcacattggcagtggctgtcctgaccctcaggcctatgctcagtccatcgcagacgcccggctcgtgtttgaaatgggcaccg
agctgggtcacaagatgcacgttctggaccttggtggtggcttccctggcacagaaggggccaaagtgagatttgaagagattgcttccgtgatca
actcagccttggacctgtacttcccagagggctgtggcgtggacatctttgctgagctggggcgctactacgtgacctcggccttcactgtggcagt
cagcatcattgccaagaaggaggttctgctagaccagcctggcagggaggaggaaaatggttccacctccaagaccatcgtgtaccaccttgatg
agggcgtgtatgggatcttcaactcagtcctgtttgacaacatctgccctacccccatcctgcagaagtctaagaaccactcaccctgctacatgtctc
tagagtccattcatttcattgccgtgtagcgctctttg
(Seq. ID. No: 22)

Fig. 7a
MAGYLSESDFVMVEEGFSTRDLLKELTLGASQATTFSHWQWLS.PSGLCSVHRRRPARV.NG
HRAGSQDARSGPWWWLPWHRRGQSEI.RDCFRDQLSLGPVLPRGLWRGHLC.AGALLRDLG
LHCGSQHHCQEGGSARPAWQGGGKWFHLQDHRVPP..GRVWDLQLSPV.QHLPYPHPAEV.E
PLTLLHVSRVHSFHCRVALF
(Seq. ID. No: 23)

Fig. 8

**SV8:**

atggctggctacctgagtgaatcggactttgtgatggtggaggagggcttcagtacccgagacctgctgaaggaactcactctgggggcctcaca
ggccaccacgaaactgccatctaacatctgctgaatggacttgacgaggtagctgccttcttcgtggctgacctgggtgccatagtgaggaagcac
ttttgctttctgaagtgcctgccacgagtccggccctttatgctgtca
agtgcaacagcagcccaggtgtgctgaaggttctggcccagctggggctgggctttagctgtgccaacaaggcagagatggagttggtccagca
tattggaatccctgccagtaagatcatctgcgccaacccctgtaagcaaattgcacagatcaaatatgctgccaagcatgggatccagctgctgagc
tttgacaatgagatggagctggcaaaggtggtaaagagccaccccagtgccaagatggttctgtgcattgctaccgatgactcccactccctgagc
tgcctgagcctaaagtttggagtgtcactgaaatcctgcagacacctgcttgaaaatgcgaagaagcaccatgtggaggtggtgggtgtgagttttc
acattggcagtggctgtcctgaccctcaggcctatgctcagtccatcgcagacgcccggctcgtgtttgaaatgggcaccgagctgggtcacaag
atgcacgttctggaccttggtggtggcttccctggcacagaaggggccaaagtgagatttgaagagattgcttccgtgatcaactcagccttggacc
tgtacttcccagagggctgtggcgtggacatctttgctgagctggggcgctactacgtgacctcggccttcactgtggcagtcagcatcattgccaa
gaaggaggttctgctagaccagcctggcagggaggaggaaaatggttccacctccaagaccatcgtgtaccaccttgatgagggcgtgtatggg
atcttcaactcagtcctgtttgacaacatctgccctacccccatcctgcagaagtctaagaaccactcacccctgctacatgtctctagagtccattcattt
cattgccgtgtagcgctctttg

(Seq. ID. No: 24)

Fig. 8a

MAGYLSESDFVMVEEGFSTRDLLKELTLGASQATTKLPSNIC.MDLTR.LPSSWLTWVP..GST
FAF.SACHESGPFMLSSATAAQVC.RFWPSWGWALAVPTRQRWSWSSILESLPVRSSAPTPVS
KLHRSNMLPSMGSSC.ALTMRWSWQRW.RATPVPRWFCALLPMTPTP.AA.A.SLECH.NPAD
TCLKMRRSTMWRWWV.VFTLAVAVLTLRPMLSPSQTPGSCLKWAPSWVTRCTFWTLVVAS
LAQKGPK.DLKRLLP.STQPWTCTSQRAVAWTSLLSWGATT.PRPSLWQSASLPRRRFC.TSLA
GRRKMVPPPRPSCTTLMRACMGSSTQSCLTTSALPPSCRSLRTTHPATCL.SPFISLPCSALL

(Seq. ID. No: 25)

Fig. 9
Exon I:

TGTGTTGCATACTTTCTAAGGCGGCGGCTGCAGCAGCGGCTCCATCCAGC
CCGTCAGCTCCTCCTGCAAGGCATGGCTGGCTACCTGAGTGAATCGGAC
TTTGTGATGGTGGAGGAGGGCTTCAGTACCCGAGACCTGCTGAAGGAAC
TCACTCTGGGGGCCTCACAGGCCACCACG
(Seq. ID. No: 26)

Exon II:
AAACTGCCATCTAACATCTGCTGAATGGACTT
(Seq. ID. No: 27)

Exon III:
GACGAGGTAGCTGCCTTCTTCGTGGCTGACCTGGGTGCCATAGTGAGGA
AGCACTTTTGCTTTCTGAAGTGCCTGCCACGAGTCCGGCCCTTTTATGCT
GTCAAGTGCAACAGCAGCCCAGGTGTGCTGAAGGTTCTGGCCCAGCTGG
GGCTGGGCTTTAGCTGTGCCAACAAG
(Seq. ID. No: 28)

Exon IV:
GCAGAGATGGAGTTGGTCCAGCATATTGGAATCCCTGCCAGTAAGATCA
TCTGCGCCAACCCCTGTAAGCAAATTGCACAGATCAAATATGCTGCCAA
GCATGGGATCCAGCTGCTGAGCTTTGACAATGAGATGGAGCTGGCAAAG
GTGGTAAAGAGCCACCCCAGTGCCAAG
(Seq. ID. No: 29)

Exon V:
GATGGTTCTGTGCATTGCTACCGATGACTCCCACTCCCTGAGCTGCCTGA
GCCTAAAGTTTGGAGTGTCACTGAAATCCTGCAGACACCTGCTTGAAAA
TGCGAAGAAGCACCATGTGGAGGTGGTGGGTGTGAG
(Seq. ID. No: 30)

Exon VI:
TTTTCACATTGGCAGTGGCTGTCCTGACCCTCAGGCCTATGCTCAGTCCA
TCGCAGACGCCCGGCTCGTGTTTGAAATGGGCACCGAGCTGGGTCACAA
GATGCACGTTCTGGACCTTGGTGGTGGCTTCCCTGGCACAGAAGGGGCC
AAAGTGAGATTTGAAGAG
(Seq. ID. No: 31)

Exon VII:

ATTGCTTCCGTGATCAACTCAGCCTTGGACCTGTACTTCCCAGAGGGCTG
TGGCGTGGACATCTTTGCTGAGCTGGGGCGCTACTACGTGACCTCGGCCT
TCACTGTGGCAGTCAGCATCATTGCCAAGAAGGAGGTTCTGCTAGACCA
GCCTGGCAGGGAGG

(Seq. ID. No: 32)

Exon VIII:

AGGAAAATGGTTCCACCTCCAAGACCATCGTGTACCACCTTGATGAGGG
CGTGTATGGGATCTTCAACTCAGTCCTGTTTGACAACATCTGCCCTACCC
CCATCCTGCAGAAG

(Seq. ID. No: 33)

Exon IX:

TCTAAGAACCACTCACCCTGCTACATGTCTCTAGAGTCCATTCATTTCATTGCCGTG**TAG**
CGCTCTTTTG

(Seq. ID. No: 34)

Exon X:

AAACCATCCACGGAGCAGCCCCTGTACAGCAGCAGCCTGTGGGGCCCGGCGGTTGATGG
CTGTGATTGCGTGGCTGAGGGCCTGTGGCTGCCGCAACTACACGTAGGGGACTGGCTGG
TCTTTGACAACATGGGCGCCTACACTGTGGGCATGGGTTCCCCCTTTTGGGGGACCCAGG
CCTGCCACATCACCTATGCCATGTCCCGGGTGGCCTG

(Seq. ID. No: 35)

Exon XI:

GGAAGCGCTGCGAAGGCAGCTGATGGCTGCAGAACAGGAGGATGACGTGGAGGGTGTG
TGCAAGCCTCTGTCCTGCGGCTGGGAGATCACAGACACCCTGTGCGTGGGCCCTGTCTTC
ACCCCAGCGAGCATCATG**TGA**GTGGGCCTCGTTCCCCCCGGAGAATCCCAGCGGGGCCT
CAGAGATGCATCTGGGAGAGGTGGGGAAGATGGCAGGCAAGGGTACCCTTGGCCAGGA
CTCTGGTGCCCACCCTGCCACCCCCGCGCTCCACCTGCAGTGTTTCTGCCCTGTAAATAG
GACCAGTCTTACACTCGCTGTAGTTCAAGTATGCAACATAAATCCTGTTCCTTCCAGCTG
TGTCTGCCTCCTCTGCAGTGCAAGGGGCCTGGTCAGCCAGGTGTGGGGGTGTTCTTGGGG
TCTCCTTTGGTCTCCTTCCCACCTTTGTAAATATAATGCAAATAAATAAATATTTAGGTTT
TTAAAAACTGCAGCGGAATCTGGCAGTTTGATTCACAAAGCAGCCTGGGCTAGGCCTGG
GGCAGGATTTCCCCATCACTCACTGATGAGCCCACACCCTCTGCTTTA

(Seq. ID. No: 36)

European Patent Office

## PARTIAL EUROPEAN SEARCH REPORT

which under Rule 45 of the European Patent Convention shall be considered, for the purposes of subsequent proceedings, as the European search report

Application Number EP 02 25 5559

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE EMBL [Online] 13 July 2001 (2001-07-13) Database accession no. BC010449 XP002223468 * the whole document * --- | 1-10 | C12N9/00 |
| P,X | DATABASE EMBL [Online] 4 October 2001 (2001-10-04) Database accession no. AY050634 XP002223469 * the whole document * --- | 1-10 | |
| P,X | DATABASE SWISSPROT [Online] 15 June 2002 (2002-06-15) Database accession no. Q96A70 XP002223470 * the whole document * --- | 1-10 | |
| P,X | DATABASE EMBL [Online] 6 February 2002 (2002-02-06) Database accession no. AX350367 XP002223471 * the whole document * ----- | 1,2,9,10 | TECHNICAL FIELDS SEARCHED (Int.Cl.7) C12N |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 4 December 2002 | Chavanne, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 02 25 5559

Although claims 3-8 are directed to a diagnostic method practised on the human/animal body (Article 52(4) EPC), the search has been carried out and based on the alleged effects of the compound/composition.

--------------------

Claim(s) not searched:
3-8

Reason for the limitation of the search (non-patentable invention(s)):

Article 52 (4) EPC - Diagnostic method practised on the human or animal body

SEQUENCE LISTING

```
<110>  Heiskala, Marja
       Christer Leander Andersson, Leif
       Fitkanen, Laura

<120>  ORNITHINE DECARBOXYLASE RELATED PROTEIN AND RELATED

<130>  CDS-239

<140>  60/311,063
<141>  2001-08-09

<160>  36

<170>  PatentIn version 3.1

<210>  1
<211>  23
<212>  DNA
<213>  Synthetic construct

<400>  1
tcagctcctc ctgcaaggca tgg                                              23


<210>  2
<211>  22
<212>  DNA
<213>  Synthetic construct

<400>  2
gaggcccact cacatgctcg ct                                               22


<210>  3
<211>  25
<212>  DNA
<213>  Synthetic construct

<400>  3
ctacacggca atgaaatgaa tggac                                            25


<210>  4
<211>  25
<212>  DNA
<213>  Synthetic construct

<400>  4
gaggcccact cacatgatgc tcgct                                            25


<210>  5
<211>  15
<212>  DNA
<213>  Synthetic construct

<400>  5
cactccctga gctgc                                                       15
```

<210> 6
<211> 16
<212> DNA
<213> Synthetic construct

<400> 6
ctgctccgtg gatggt 16

<210> 7
<211> 18
<212> DNA
<213> Synthetic construct

<400> 7
cttttgcttt ctgaagtg 18

<210> 8
<211> 18
<212> DNA
<213> Synthetic construct

<400> 8
aaagcagtct gtcgtctc 18

<210> 9
<211> 17
<212> DNA
<213> Synthetic construct

<400> 9
gcgctcaaca atccgat 17

<210> 10
<211> 1383
<212> DNA
<213> Human

<400> 10
atggctggct acctgagtga atcggacttt gtgatggtgg aggagggctt cagtacccga 60

gacctgctga aggaactcac tctgggggcc tcacaggcca ccacggacga ggtagctgcc 120

ttcttcgtgg ctgacctggg tgccatagtg aggaagcact tttgctttct gaagtgcctg 180

ccacgagtcc ggcccttta tgctgtcaag tgcaacagca gcccaggtgt gctgaaggtt 240

ctggcccagc tggggctggg ctttagctgt gccaacaagg cagagatgga gttggtccag 300

catattggaa tccctgccag taagatcatc tgcgccaacc cctgtaagca aattgcacag 360

atcaaatatg ctgccaagca tgggatccag ctgctgagct ttgacaatga gatggagctg 420

gcaaaggtgg taaagagcca ccccagtgcc aagatggttc tgtgcattgc taccgatgac 480

tcccactccc tgagctgcct gagcctaaag tttggagtgt cactgaaatc ctgcagacac 540

ctgcttgaaa atgcgaagaa gcaccatgtg gaggtggtgg gtgtgagttt tcacattggc 600
agtggctgtc ctgaccctca ggcctatgct cagtccatcg cagacgcccg gctcgtgttt 660
gaaatgggca ccgagctggg tcacaagatg cacgttctgg accttggtgg tggcttccct 720
ggcacagaag ggggccaaagt gagatttgaa gagattgctt ccgtgatcaa ctcagccttg 780
gacctgtact tcccagaggg ctgtggcgtg gacatctttg ctgagctggg gcgctactac 840
gtgacctcgg ccttcactgt ggcagtcagc atcattgcca agaaggaggt tctgctagac 900
cagcctggca gggaggagga aaatggttcc acctccaaga ccatcgtgta ccaccttgat 960
gagggcgtgt atgggatctt caactcagtc ctgtttgaca acatctgccc taccccccatc 1020
ctgcagaaga aaccatccac ggagcagccc ctgtacagca gcagcctgtg ggcccggcg 1080
gttgatggct gtgattgcgt ggctgagggc ctgtggctgc cgcaactaca cgtaggggac 1140
tggctggtct ttgacaacat gggcgcctac actgtgggca tgggttcccc cttttgggg 1200
acccaggcct gccacatcac ctatgccatg tcccgggtgg cctgggaagc gctgcgaagg 1260
cagctgatgg ctgcagaaca ggaggatgac gtggagggtg tgtgcaagcc tctgtcctgc 1320
ggctgggaga tcacagacac cctgtgcgtg ggccctgtct tcaccccagc gagcatcatg 1380
tga 1383

<210> 11
<211> 460
<212> PRT
<213> Human

<400> 11

Met Ala Gly Tyr Leu Ser Glu Ser Asp Phe Val Met Val Glu Glu Gly
1 5 10 15

Phe Ser Thr Arg Asp Leu Leu Lys Glu Leu Thr Leu Gly Ala Ser Gln
20 25 30

Ala Thr Thr Asp Glu Val Ala Ala Phe Phe Val Ala Asp Leu Gly Ala
35 40 45

Ile Val Arg Lys His Phe Cys Phe Leu Lys Cys Leu Pro Arg Val Arg
50 55 60

Pro Phe Tyr Ala Val Lys Cys Asn Ser Ser Pro Gly Val Leu Lys Val
65 70 75 80

Leu Ala Gln Leu Gly Leu Gly Phe Ser Cys Ala Asn Lys Ala Glu Met
85 90 95

Glu Leu Val Gln His Ile Gly Ile Pro Ala Ser Lys Ile Ile Cys Ala
100 105 110

Asn Pro Cys Lys Gln Ile Ala Gln Ile Lys Tyr Ala Ala Lys His Gly
115 120 125

Ile Gln Leu Leu Ser Phe Asp Asn Glu Met Glu Leu Ala Lys Val Val
130 135 140

Lys Ser His Pro Ser Ala Lys Met Val Leu Cys Ile Ala Thr Asp Asp
145 150 155 160

Ser His Ser Leu Ser Cys Leu Ser Leu Lys Phe Gly Val Ser Leu Lys
165 170 175

Ser Cys Arg His Leu Leu Glu Asn Ala Lys Lys His His Val Glu Val
180 185 190

Val Gly Val Ser Phe His Ile Gly Ser Gly Cys Pro Asp Pro Gln Ala
195 200 205

Tyr Ala Gln Ser Ile Ala Asp Ala Arg Leu Val Phe Glu Met Gly Thr
210 215 220

Glu Leu Gly His Lys Met His Val Leu Asp Leu Gly Gly Gly Phe Pro
225 230 235 240

Gly Thr Glu Gly Ala Lys Val Arg Phe Glu Glu Ile Ala Ser Val Ile
245 250 255

Asn Ser Ala Leu Asp Leu Tyr Phe Pro Glu Gly Cys Gly Val Asp Ile
260 265 270

Phe Ala Glu Leu Gly Arg Tyr Tyr Val Thr Ser Ala Phe Thr Val Ala
275 280 285

Val Ser Ile Ile Ala Lys Lys Glu Val Leu Leu Asp Gln Pro Gly Arg
290 295 300

Glu Glu Glu Asn Gly Ser Thr Ser Lys Thr Ile Val Tyr His Leu Asp
305 310 315 320

Glu Gly Val Tyr Gly Ile Phe Asn Ser Val Leu Phe Asp Asn Ile Cys
325 330 335

```
Pro Thr Pro Ile Leu Gln Lys Lys Pro Ser Thr Glu Gln Pro Leu Tyr
            340                 345                 350

Ser Ser Ser Leu Trp Gly Pro Ala Val Asp Gly Cys Asp Cys Val Ala
        355                 360                 365

Glu Gly Leu Trp Leu Pro Gln Leu His Val Gly Asp Trp Leu Val Phe
    370                 375                 380

Asp Asn Met Gly Ala Tyr Thr Val Gly Met Gly Ser Pro Phe Trp Gly
385                 390                 395                 400

Thr Gln Ala Cys His Ile Thr Tyr Ala Met Ser Arg Val Ala Trp Glu
                405                 410                 415

Ala Leu Arg Arg Gln Leu Met Ala Ala Glu Gln Glu Asp Asp Val Glu
            420                 425                 430

Gly Val Cys Lys Pro Leu Ser Cys Gly Trp Glu Ile Thr Asp Thr Leu
        435                 440                 445

Cys Val Gly Pro Val Phe Thr Pro Ala Ser Ile Met
    450                 455                 460


<210>  12
<211>  1443
<212>  DNA
<213>  Human

<400>  12
atggctggct acctgagtga atcggacttt gtgatggtgg aggagggctt cagtacccga      60

gacctgctga aggaactcac tctgggggcc tcacaggcca ccacggacga ggtagctgcc     120

ttcttcgtgg ctgacctggg tgccatagtg aggaagcact tttgctttct gaagtgcctg     180

ccacgagtcc ggccctttta tgctgtcaag tgcaacagca gcccaggtgt gctgaaggtt     240

ctggcccagc tggggctggg ctttagctgt gccaacaagg cagagatgga gttggtccag     300

catattggaa tccctgccag taagatcatc tgcgccaacc cctgtaagca aattgcacag     360

atcaaatatg ctgccaagca tgggatccag ctgctgagct ttgacaatga gatggagctg     420

gcaaaggtgg taaagagcca ccccagtgcc aagatggttc tgtgcattgc taccgatgac     480

tcccactccc tgagctgcct gagcctaaag tttggagtgt cactgaaatc ctgcagacac     540

ctgcttgaaa atgcgaagaa gcaccatgtg gaggtggtgg gtgtgagttt tcacattggc     600

agtggctgtc ctgaccctca ggcctatgct cagtccatcg cagacgcccg gctcgtgttt     660

gaaatgggca ccgagctggg tcacaagatg cacgttctgg accttggtgg tggcttccct     720
```

ggcacagaag gggccaaagt gagatttgaa gagattgctt ccgtgatcaa ctcagccttg 780

gacctgtact tcccagaggg ctgtggcgtg gacatctttg ctgagctggg gcgctactac 840

gtgacctcgg ccttcactgt ggcagtcagc atcattgcca agaaggaggt tctgctagac 900

cagcctggca gggaggcccc actaccaccc cctcacattg ctacttgtgc tgcctctgaa 960

ccctcccctc ctgcagagga aaatggttcc acctccaaga ccatcgtgta ccaccttgat 1020

gagggcgtgt atgggatctt caactcagtc ctgtttgaca acatctgccc taccccccatc 1080

ctgcagaaga aaccatccac ggagcagccc ctgtacagca gcagcctgtg gggcccggcg 1140

gttgatggct gtgattgcgt ggctgagggc ctgtggctgc cgcaactaca cgtaggggac 1200

tggctggtct ttgacaacat gggcgcctac actgtgggca tgggttcccc cttttggggg 1260

acccaggcct gccacatcac ctatgccatg tcccgggtgg cctgggaagc gctgcgaagg 1320

cagctgatgg ctgcagaaca ggaggatgac gtggagggtg tgtgcaagcc tctgtcctgc 1380

ggctgggaga tcacagacac cctgtgcgtg ggccctgtct tcaccccagc gagcatcatg 1440

tga 1443

<210> 13
<211> 480
<212> PRT
<213> Human

<400> 13

Met Ala Gly Tyr Leu Ser Glu Ser Asp Phe Val Met Val Glu Glu Gly
1 5 10 15

Phe Ser Thr Arg Asp Leu Leu Lys Glu Leu Thr Leu Gly Ala Ser Gln
20 25 30

Ala Thr Thr Asp Glu Val Ala Ala Phe Phe Val Ala Asp Leu Gly Ala
35 40 45

Ile Val Arg Lys His Phe Cys Phe Leu Lys Cys Leu Pro Arg Val Arg
50 55 60

Pro Phe Tyr Ala Val Lys Cys Asn Ser Ser Pro Gly Val Leu Lys Val
65 70 75 80

Leu Ala Gln Leu Gly Leu Gly Phe Ser Cys Ala Asn Lys Ala Glu Met
85 90 95

Glu Leu Val Gln His Ile Gly Ile Pro Ala Ser Lys Ile Ile Cys Ala
100 105 110

```
Asn Pro Cys Lys Gln Ile Ala Gln Ile Lys Tyr Ala Ala Lys His Gly
        115                 120                 125

Ile Gln Leu Leu Ser Phe Asp Asn Glu Met Glu Leu Ala Lys Val Val
    130                 135                 140

Lys Ser His Pro Ser Ala Lys Met Val Leu Cys Ile Ala Thr Asp Asp
145                 150                 155                 160

Ser His Ser Leu Ser Cys Leu Ser Leu Lys Phe Gly Val Ser Leu Lys
                165                 170                 175

Ser Cys Arg His Leu Leu Glu Asn Ala Lys Lys His His Val Glu Val
            180                 185                 190

Val Gly Val Ser Phe His Ile Gly Ser Gly Cys Pro Asp Pro Gln Ala
        195                 200                 205

Tyr Ala Gln Ser Ile Ala Asp Ala Arg Leu Val Phe Glu Met Gly Thr
    210                 215                 220

Glu Leu Gly His Lys Met His Val Leu Asp Leu Gly Gly Gly Phe Pro
225                 230                 235                 240

Gly Thr Glu Gly Ala Lys Val Arg Phe Glu Glu Ile Ala Ser Val Ile
                245                 250                 255

Asn Ser Ala Leu Asp Leu Tyr Phe Pro Glu Gly Cys Gly Val Asp Ile
            260                 265                 270

Phe Ala Glu Leu Gly Arg Tyr Tyr Val Thr Ser Ala Phe Thr Val Ala
        275                 280                 285

Val Ser Ile Ile Ala Lys Lys Glu Val Leu Leu Asp Gln Pro Gly Arg
    290                 295                 300

Glu Ala Pro Leu Pro Pro Pro His Ile Ala Thr Cys Ala Ala Ser Glu
305                 310                 315                 320

Pro Ser Pro Pro Ala Glu Glu Asn Gly Ser Thr Ser Lys Thr Ile Val
                325                 330                 335

Tyr His Leu Asp Glu Gly Val Tyr Gly Ile Phe Asn Ser Val Leu Phe
            340                 345                 350
```

Asp Asn Ile Cys Pro Thr Pro Ile Leu Gln Lys Lys Pro Ser Thr Glu
355 360 365

Gln Pro Leu Tyr Ser Ser Ser Leu Trp Gly Pro Ala Val Asp Gly Cys
370 375 380

Asp Cys Val Ala Glu Gly Leu Trp Leu Pro Gln Leu His Val Gly Asp
385 390 395 400

Trp Leu Val Phe Asp Asn Met Gly Ala Tyr Thr Val Gly Met Gly Ser
405 410 415

Pro Phe Trp Gly Thr Gln Ala Cys His Ile Thr Tyr Ala Met Ser Arg
420 425 430

Val Ala Trp Glu Ala Leu Arg Arg Gln Leu Met Ala Ala Glu Gln Glu
435 440 445

Asp Asp Val Glu Gly Val Cys Lys Pro Leu Ser Cys Gly Trp Glu Ile
450 455 460

Thr Asp Thr Leu Cys Val Gly Pro Val Phe Thr Pro Ala Ser Ile Met
465 470 475 480

<210> 14
<211> 1089
<212> DNA
<213> Human

<400> 14
atggctggct acctgagtga atcggacttt gtgatggtgg aggagggctt cagtacccga 60
gacctgctga aggaactcac tctgggggcc tcacaggcca ccacggacga ggtagctgcc 120
ttcttcgtgg ctgacctggg tgccatagtg aggaagcact tttgctttct gaagtgcctg 180
ccacgagtcc ggcccttttta tgctgtcaag tgcaacagca gcccaggtgt gctgaaggtt 240
ctggcccagc tggggctggg ctttagctgt gccaacaagg cagagatgga gttggtccag 300
catattggaa tccctgccag taagatcatc tgcgccaacc cctgtaagca aattgcacag 360
atcaaatatg ctgccaagca tgggatccag ctgctgagct ttgacaatga gatggagctg 420
gcaaaggtgg taaagagcca ccccagtgcc aagatggttc tgtgcattgc taccgatgac 480
tcccactccc tgagctgcct gagcctaaag tttggagtgt cactgaaatc ctgcagacac 540
ctgcttgaaa atgcgaagaa gcaccatgtg gaggtggtgg gtgtgagttt tcacattggc 600
agtggctgtc ctgaccctca ggcctatgct cagtccatcg cagacgcccg gctcgtgttt 660

gaaatgggca ccgagctggg tcacaagatg cacgttctgg accttggtgg tggcttccct 720

ggcacagaag gggccaaagt gagatttgaa gagattgctt ccgtgatcaa ctcagccttg 780

gacctgtact tcccagaggg ctgtggcgtg gacatctttg ctgagctggg gcgctactac 840

gtgacctcgg ccttcactgt ggcagtcagc atcattgcca agaaggaggt tctgctagac 900

cagcctggca gggaggagga aaatggttcc acctccaaga ccatcgtgta ccaccttgat 960

gagggcgtgt atgggatctt caactcagtc ctgtttgaca acatctgccc tacccccatc 1020

ctgcagaagt ctaagaacca ctcaccctgc tacatgtctc tagagtccat tcatttcatt 1080

gccgtgtag 1089

<210> 15
<211> 362
<212> PRT
<213> Human

<400> 15

Met Ala Gly Tyr Leu Ser Glu Ser Asp Phe Val Met Val Glu Glu Gly
1 5 10 15

Phe Ser Thr Arg Asp Leu Leu Lys Glu Leu Thr Leu Gly Ala Ser Gln
20 25 30

Ala Thr Thr Asp Glu Val Ala Ala Phe Phe Val Ala Asp Leu Gly Ala
35 40 45

Ile Val Arg Lys His Phe Cys Phe Leu Lys Cys Leu Pro Arg Val Arg
50 55 60

Pro Phe Tyr Ala Val Lys Cys Asn Ser Ser Pro Gly Val Leu Lys Val
65 70 75 80

Leu Ala Gln Leu Gly Leu Gly Phe Ser Cys Ala Asn Lys Ala Glu Met
85 90 95

Glu Leu Val Gln His Ile Gly Ile Pro Ala Ser Lys Ile Ile Cys Ala
100 105 110

Asn Pro Cys Lys Gln Ile Ala Gln Ile Lys Tyr Ala Ala Lys His Gly
115 120 125

Ile Gln Leu Leu Ser Phe Asp Asn Glu Met Glu Leu Ala Lys Val Val
130 135 140

Lys Ser His Pro Ser Ala Lys Met Val Leu Cys Ile Ala Thr Asp Asp

145 150 155 160

Ser His Ser Leu Ser Cys Leu Ser Leu Lys Phe Gly Val Ser Leu Lys
165 170 175

Ser Cys Arg His Leu Leu Glu Asn Ala Lys Lys His His Val Glu Val
180 185 190

Val Gly Val Ser Phe His Ile Gly Ser Gly Cys Pro Asp Pro Gln Ala
195 200 205

Tyr Ala Gln Ser Ile Ala Asp Ala Arg Leu Val Phe Glu Met Gly Thr
210 215 220

Glu Leu Gly His Lys Met His Val Leu Asp Leu Gly Gly Gly Phe Pro
225 230 235 240

Gly Thr Glu Gly Ala Lys Val Arg Phe Glu Glu Ile Ala Ser Val Ile
245 250 255

Asn Ser Ala Leu Asp Leu Tyr Phe Pro Glu Gly Cys Gly Val Asp Ile
260 265 270

Phe Ala Glu Leu Gly Arg Tyr Tyr Val Thr Ser Ala Phe Thr Val Ala
275 280 285

Val Ser Ile Ile Ala Lys Lys Glu Val Leu Leu Asp Gln Pro Gly Arg
290 295 300

Glu Glu Glu Asn Gly Ser Thr Ser Lys Thr Ile Val Tyr His Leu Asp
305 310 315 320

Glu Gly Val Tyr Gly Ile Phe Asn Ser Val Leu Phe Asp Asn Ile Cys
325 330 335

Pro Thr Pro Ile Leu Gln Lys Ser Lys Asn His Ser Pro Cys Tyr Met
340 345 350

Ser Leu Glu Ser Ile His Phe Ile Ala Val
355 360

<210> 16
<211> 1125
<212> DNA
<213> Human

<400> 16

```
atggctggct acctgagtga atcggacttt gtgatggtgg aggagggctt cagtacccga      60
gacctgctga aggaactcac tctgggggcc tcacaggcca ccacggacga ggtagctgcc     120
ttcttcgtgg ctgacctggg tgccatagtg aggaagcact tttgcttttct gaagtgcctg    180
ccacgagtcc ggccctttta tgctgtcaag tgcaacagca gcccaggtgt gctgaaggtt     240
ctggcccagc tggggctggg ctttagctgt gccaacaagg cagagatgga gttggtccag     300
catattggaa tccctgccag taagatcatc tgcgccaacc cctgtaagca aattgcacag     360
atcaaatatg ctgccaagca tgggatccag ctgctgagct ttgacaatga gatggagctg     420
gcaaaggtgg taaagagcca ccccagtgcc aagtttgtcc agcagagggg cactgcgtgt     480
ctcatcagga tggttctgtg cattgctacc gatgactccc actccctgag ctgcctgagc     540
ctaaagtttg gagtgtcact gaaatcctgc agacacctgc ttgaaaatgc gaagaagcac     600
catgtggagg tggtgggtgt gagttttcac attggcagtg gctgtcctga ccctcaggcc     660
tatgctcagt ccatcgcaga cgcccggctc gtgtttgaaa tgggcaccga gctgggtcac     720
aagatgcacg ttctggacct tggtggtggc ttccctggca cagaaggggc caaagtgaga     780
tttgaagaga ttgcttccgt gatcaactca gccttggacc tgtacttccc agagggctgt     840
ggcgtggaca tctttgctga gctggggcgc tactacgtga cctcggcctt cactgtggca     900
gtcagcatca ttgccaagaa ggaggttctg ctagaccagc ctggcaggga ggaggaaaat     960
ggttccacct ccaagaccat cgtgtaccac cttgatgagg gcgtgtatgg gatcttcaac    1020
tcagtcctgt ttgacaacat ctgccctacc cccatcctgc agaagtctaa gaaccactca    1080
ccctgctaca tgtctctaga gtccattcat ttcattgccg tgtag                   1125
```

<210> 17
<211> 374
<212> PRT
<213> Human

<400> 17

```
Met Ala Gly Tyr Leu Ser Glu Ser Asp Phe Val Met Val Glu Glu Gly
1               5                   10                  15

Phe Ser Thr Arg Asp Leu Leu Lys Glu Leu Thr Leu Gly Ala Ser Gln
            20                  25                  30

Ala Thr Thr Asp Glu Val Ala Ala Phe Phe Val Ala Asp Leu Gly Ala
        35                  40                  45

Ile Val Arg Lys His Phe Cys Phe Leu Lys Cys Leu Pro Arg Val Arg
    50                  55                  60
```

```
Pro Phe Tyr Ala Val Lys Cys Asn Ser Ser Pro Gly Val Leu Lys Val
65                  70                  75                  80

Leu Ala Gln Leu Gly Leu Gly Phe Ser Cys Ala Asn Lys Ala Glu Met
                85                  90                  95

Glu Leu Val Gln His Ile Gly Ile Pro Ala Ser Lys Ile Ile Cys Ala
            100                 105                 110

Asn Pro Cys Lys Gln Ile Ala Gln Ile Lys Tyr Ala Ala Lys His Gly
        115                 120                 125

Ile Gln Leu Leu Ser Phe Asp Asn Glu Met Glu Leu Ala Lys Val Val
    130                 135                 140

Lys Ser His Pro Ser Ala Lys Phe Val Gln Gln Arg Gly Thr Ala Cys
145                 150                 155                 160

Leu Ile Arg Met Val Leu Cys Ile Ala Thr Asp Asp Ser His Ser Leu
                165                 170                 175

Ser Cys Leu Ser Leu Lys Phe Gly Val Ser Leu Lys Ser Cys Arg His
            180                 185                 190

Leu Leu Glu Asn Ala Lys Lys His His Val Glu Val Val Gly Val Ser
        195                 200                 205

Phe His Ile Gly Ser Gly Cys Pro Asp Pro Gln Ala Tyr Ala Gln Ser
    210                 215                 220

Ile Ala Asp Ala Arg Leu Val Phe Glu Met Gly Thr Glu Leu Gly His
225                 230                 235                 240

Lys Met His Val Leu Asp Leu Gly Gly Gly Phe Pro Gly Thr Glu Gly
                245                 250                 255

Ala Lys Val Arg Phe Glu Glu Ile Ala Ser Val Ile Asn Ser Ala Leu
            260                 265                 270

Asp Leu Tyr Phe Pro Glu Gly Cys Gly Val Asp Ile Phe Ala Glu Leu
        275                 280                 285

Gly Arg Tyr Tyr Val Thr Ser Ala Phe Thr Val Ala Val Ser Ile Ile
    290                 295                 300
```

Ala Lys Lys Glu Val Leu Leu Asp Gln Pro Gly Arg Glu Glu Glu Asn
305 310 315 320

Gly Ser Thr Ser Lys Thr Ile Val Tyr His Leu Asp Glu Gly Val Tyr
325 330 335

Gly Ile Phe Asn Ser Val Leu Phe Asp Asn Ile Cys Pro Thr Pro Ile
340 345 350

Leu Gln Lys Ser Lys Asn His Ser Pro Cys Tyr Met Ser Leu Glu Ser
355 360 365

Ile His Phe Ile Ala Val
370

<210> 18
<211> 926
<212> DNA
<213> Human

<400> 18
atggctggct acctgagtga atcggacttt gtgatggtgg aggagggctt cagtacccga 60

gacctgctga aggaactcac tctgggggcc tcacaggcca ccacggacga ggtagctgcc 120

ttcttcgtgg ctgacctggg tgccatagtg aggaagcact tttgctttct gaagtgcctg 180

ccacgagtcc ggccctttta tgctgtcaag tgcaacagca gcccaggtgt gctgaaggtt 240

ctggcccagc tggggctggg ctttagctgt gccaacaagg atggttctgt gcattgctac 300

cgatgactcc cactccctga gctgcctgag cctaaagttt ggagtgtcac tgaaatcctg 360

cagacacctg cttgaaaatg cgaagaagca ccatgtggag gtggtgggtg tgagttttca 420

cattggcagt ggctgtcctg accctcaggc ctatgctcag tccatcgcag acgcccggct 480

cgtgtttgaa atgggcaccg agctgggtca caagatgcac gttctggacc ttggtggtgg 540

cttccctggc acagaagggg ccaaagtgag atttgaagag attgcttccg tgatcaactc 600

agccttggac ctgtacttcc cagagggctg tggcgtggac atctttgctg agctggggcg 660

ctactacgtg acctcggcct tcactgtggc agtcagcatc attgccaaga aggaggttct 720

gctagaccag cctggcaggg aggaggaaaa tggttccacc tccaagacca tcgtgtacca 780

ccttgatgag ggcgtgtatg ggatcttcaa ctcagtcctg tttgacaaca tctgccctac 840

ccccatcctg cagaagtcta agaaccactc accctgctac atgtctctag agtccattca 900

tttcattgcc gtgtagcgct cttttg 926

<210> 19
<211> 298

<212> PRT
<213> Human

<400> 19

```
Met Ala Gly Tyr Leu Ser Glu Ser Asp Phe Val Met Val Glu Glu Gly
1               5                   10                  15

Phe Ser Thr Arg Asp Leu Leu Lys Glu Leu Thr Leu Gly Ala Ser Gln
            20                  25                  30

Ala Thr Thr Asp Glu Val Ala Ala Phe Phe Val Ala Asp Leu Gly Ala
        35                  40                  45

Ile Val Arg Lys His Phe Cys Phe Leu Lys Cys Leu Pro Arg Val Arg
    50                  55                  60

Pro Phe Tyr Ala Val Lys Cys Asn Ser Ser Pro Gly Val Leu Lys Val
65                  70                  75                  80

Leu Ala Gln Leu Gly Leu Gly Phe Ser Cys Ala Asn Lys Asp Gly Ser
                85                  90                  95

Val His Cys Tyr Arg Leu Pro Leu Pro Glu Leu Pro Glu Pro Lys Val
            100                 105                 110

Trp Ser Val Thr Glu Ile Leu Gln Thr Pro Ala Lys Cys Glu Glu Ala
        115                 120                 125

Pro Cys Gly Gly Gly Gly Cys Glu Phe Ser His Trp Gln Trp Leu Ser
    130                 135                 140

Pro Ser Gly Leu Cys Ser Val His Arg Arg Arg Pro Ala Arg Val Asn
145                 150                 155                 160

Gly His Arg Ala Gly Ser Gln Asp Ala Arg Ser Gly Pro Trp Trp Trp
                165                 170                 175

Leu Pro Trp His Arg Arg Gly Gln Ser Glu Ile Arg Asp Cys Phe Arg
            180                 185                 190

Asp Gln Leu Ser Leu Gly Pro Val Leu Pro Arg Gly Leu Trp Arg Gly
        195                 200                 205

His Leu Cys Ala Gly Ala Leu Leu Arg Asp Leu Gly Leu His Cys Gly
    210                 215                 220
```

Ser Gln His His Cys Gln Glu Gly Gly Ser Ala Arg Pro Ala Trp Gln
225 230 235 240

Gly Gly Gly Lys Trp Phe His Leu Gln Asp His Arg Val Pro Pro Gly
245 250 255

Arg Val Trp Asp Leu Gln Leu Ser Pro Val Gln His Leu Pro Tyr Pro
260 265 270

His Pro Ala Glu Val Glu Pro Leu Thr Leu Leu His Val Ser Arg Val
275 280 285

His Ser Phe His Cys Arg Val Ala Leu Phe
290 295

<210> 20
<211> 615
<212> DNA
<213> Human

<400> 20
atggctggct acctgagtga atcggacttt gtgatggtgg aggagggctt cagtaccccga 60
gacctgctga aggaactcac tctgggggcc tcacaggcca ccacggacga ggtagctgcc 120
ttcttcgtgg ctgacctggg tgccatagtg aggaagcact tttgctttct gaagtgcctg 180
ccacgagtcc ggccctttta tgctgtcaag tgcaacagca gcccaggtgt gctgaaggtt 240
ctggcccagc tggggctggg ctttagctgt gccaacaaga ttgcttccgt gatcaactca 300
gccttggacc tgtacttccc agagggctgt ggcgtggaca tctttgctga gctggggcgc 360
tactacgtga cctcggcctt cactgtggca gtcagcatca ttgccaagaa ggaggttctg 420
ctagaccagc ctggcaggga ggaggaaaat ggttccacct ccaagaccat cgtgtaccac 480
cttgatgagg gcgtgtatgg gatcttcaac tcagtcctgt ttgacaacat ctgccctacc 540
cccatcctgc agaagtctaa gaaccactca ccctgctaca tgtctctaga gtccattcat 600
ttcattgccg tgtag 615

<210> 21
<211> 204
<212> PRT
<213> Human

<400> 21

Met Ala Gly Tyr Leu Ser Glu Ser Asp Phe Val Met Val Glu Glu Gly
1 5 10 15

Phe Ser Thr Arg Asp Leu Leu Lys Glu Leu Thr Leu Gly Ala Ser Gln

```
            20                  25                  30

Ala Thr Thr Asp Glu Val Ala Ala Phe Phe Val Ala Asp Leu Gly Ala
        35                  40                  45

Ile Val Arg Lys His Phe Cys Phe Leu Lys Cys Leu Pro Arg Val Arg
    50                  55                  60

Pro Phe Tyr Ala Val Lys Cys Asn Ser Ser Pro Gly Val Leu Lys Val
65                  70                  75                  80

Leu Ala Gln Leu Gly Leu Gly Phe Ser Cys Ala Asn Lys Ile Ala Ser
                85                  90                  95

Val Ile Asn Ser Ala Leu Asp Leu Tyr Phe Pro Glu Gly Cys Gly Val
            100                 105                 110

Asp Ile Phe Ala Glu Leu Gly Arg Tyr Tyr Val Thr Ser Ala Phe Thr
        115                 120                 125

Val Ala Val Ser Ile Ile Ala Lys Lys Glu Val Leu Leu Asp Gln Pro
    130                 135                 140

Gly Arg Glu Glu Glu Asn Gly Ser Thr Ser Lys Thr Ile Val Tyr His
145                 150                 155                 160

Leu Asp Glu Gly Val Tyr Gly Ile Phe Asn Ser Val Leu Phe Asp Asn
                165                 170                 175

Ile Cys Pro Thr Pro Ile Leu Gln Lys Ser Lys Asn His Ser Pro Cys
            180                 185                 190

Tyr Met Ser Leu Glu Ser Ile His Phe Ile Ala Val
        195                 200


<210>  22
<211>  617
<212>  DNA
<213>  Human

<400>  22
atggctggct acctgagtga atcggacttt gtgatggtgg aggagggctt cagtacccga      60

gacctgctga aggaactcac tctgggggcc tcacaggcca ccacgttttc acattggcag     120

tggctgtcct gaccctcagg cctatgctca gtccatcgca gacgccccggc tcgtgtttga     180

aatgggcacc gagctgggtc acaagatgca cgttctggac cttggtggtg gcttccctgg     240
```

```
cacagaaggg gccaaagtga gatttgaaga gattgcttcc gtgatcaact cagccttgga    300
cctgtacttc ccagagggct gtggcgtgga catctttgct gagctggggc gctactacgt    360
gacctcggcc ttcactgtgg cagtcagcat cattgccaag aaggaggttc tgctagacca    420
gcctggcagg gaggaggaaa atggttccac ctccaagacc atcgtgtacc accttgatga    480
gggcgtgtat gggatcttca actcagtcct gtttgacaac atctgcccta cccccatcct    540
gcagaagtct aagaaccact caccctgcta catgtctcta gagtccattc atttcattgc    600
cgtgtagcgc tcttttg                                                  617
```

<210> 23
<211> 197
<212> PRT
<213> Human

<400> 23

```
Met Ala Gly Tyr Leu Ser Glu Ser Asp Phe Val Met Val Glu Glu Gly
1               5                   10                  15

Phe Ser Thr Arg Asp Leu Leu Lys Glu Leu Thr Leu Gly Ala Ser Gln
            20                  25                  30

Ala Thr Thr Phe Ser His Trp Gln Trp Leu Ser Pro Ser Gly Leu Cys
        35                  40                  45

Ser Val His Arg Arg Arg Pro Ala Arg Val Asn Gly His Arg Ala Gly
    50                  55                  60

Ser Gln Asp Ala Arg Ser Gly Pro Trp Trp Trp Leu Pro Trp His Arg
65                  70                  75                  80

Arg Gly Gln Ser Glu Ile Arg Asp Cys Phe Arg Asp Gln Leu Ser Leu
                85                  90                  95

Gly Pro Val Leu Pro Arg Gly Leu Trp Arg Gly His Leu Cys Ala Gly
            100                 105                 110

Ala Leu Leu Arg Asp Leu Gly Leu His Cys Gly Ser Gln His His Cys
        115                 120                 125

Gln Glu Gly Gly Ser Ala Arg Pro Ala Trp Gln Gly Gly Gly Lys Trp
    130                 135                 140

Phe His Leu Gln Asp His Arg Val Pro Pro Gly Arg Val Trp Asp Leu
145                 150                 155                 160
```

Gln Leu Ser Pro Val Gln His Leu Pro Tyr Pro His Pro Ala Glu Val
165 170 175

Glu Pro Leu Thr Leu Leu His Val Ser Arg Val His Ser Phe His Cys
180 185 190

Arg Val Ala Leu Phe
195

<210> 24
<211> 1131
<212> DNA
<213> Human

<400> 24
atggctggct acctgagtga atcggacttt gtgatggtgg aggagggctt cagtacccga 60
gacctgctga aggaactcac tctgggggcc tcacaggcca ccacgaaact gccatctaac 120
atctgctgaa tggacttgac gaggtagctg ccttcttcgt ggctgacctg ggtgccatag 180
tgaggaagca cttttgcttt ctgaagtgcc tgccacgagt ccggcccttt tatgctgtca 240
agtgcaacag cagcccaggt gtgctgaagg ttctggccca gctggggctg ggctttagct 300
gtgccaacaa ggcagagatg gagttggtcc agcatattgg aatccctgcc agtaagatca 360
tctgcgccaa cccctgtaag caaattgcac agatcaaata tgctgccaag catgggatcc 420
agctgctgag ctttgacaat gagatggagc tggcaaaggt ggtaaagagc caccccagtg 480
ccaagatggt tctgtgcatt gctaccgatg actcccactc cctgagctgc ctgagcctaa 540
agtttggagt gtcactgaaa tcctgcagac acctgcttga aaatgcgaag aagcaccatg 600
tggaggtggt gggtgtgagt tttcacattg gcagtggctg tcctgaccct caggcctatg 660
ctcagtccat cgcagacgcc cggctcgtgt ttgaaatggg caccgagctg ggtcacaaga 720
tgcacgttct ggaccttggt ggtggcttcc ctggcacaga aggggccaaa gtgagatttg 780
aagagattgc ttccgtgatc aactcagcct tggacctgta cttcccagag ggctgtggcg 840
tggacatctt tgctgagctg gggcgctact acgtgacctc ggccttcact gtggcagtca 900
gcatcattgc caagaaggag gttctgctag accagcctgg cagggaggag gaaaatggtt 960
ccacctccaa gaccatcgtg taccaccttg atgagggcgt gtatgggatc ttcaactcag 1020
tcctgtttga caacatctgc cctaccccca tcctgcagaa gtctaagaac cactcacccct 1080
gctacatgtc tctagagtcc attcatttca ttgccgtgta gcgctctttt g 1131

<210> 25
<211> 359
<212> PRT

<213> Human

<400> 25

Met Ala Gly Tyr Leu Ser Glu Ser Asp Phe Val Met Val Glu Glu Gly
1 5 10 15

Phe Ser Thr Arg Asp Leu Leu Lys Glu Leu Thr Leu Gly Ala Ser Gln
20 25 30

Ala Thr Thr Lys Leu Pro Ser Asn Ile Cys Met Asp Leu Thr Arg Leu
35 40 45

Pro Ser Ser Trp Leu Thr Trp Val Pro Gly Ser Thr Phe Ala Phe Ser
50 55 60

Ala Cys His Glu Ser Gly Pro Phe Met Leu Ser Ser Ala Thr Ala Ala
65 70 75 80

Gln Val Cys Arg Phe Trp Pro Ser Trp Gly Trp Ala Leu Ala Val Pro
85 90 95

Thr Arg Gln Arg Trp Ser Trp Ser Ser Ile Leu Glu Ser Leu Pro Val
100 105 110

Arg Ser Ser Ala Pro Thr Pro Val Ser Lys Leu His Arg Ser Asn Met
115 120 125

Leu Pro Ser Met Gly Ser Ser Cys Ala Leu Thr Met Arg Trp Ser Trp
130 135 140

Gln Arg Trp Arg Ala Thr Pro Val Pro Arg Trp Phe Cys Ala Leu Leu
145 150 155 160

Pro Met Thr Pro Thr Pro Ala Ala Ala Ser Leu Glu Cys His Asn Pro
165 170 175

Ala Asp Thr Cys Leu Lys Met Arg Arg Ser Thr Met Trp Arg Trp Trp
180 185 190

Val Val Phe Thr Leu Ala Val Ala Val Leu Thr Leu Arg Pro Met Leu
195 200 205

Ser Pro Ser Gln Thr Pro Gly Ser Cys Leu Lys Trp Ala Pro Ser Trp
210 215 220

Val Thr Arg Cys Thr Phe Trp Thr Leu Val Val Ala Ser Leu Ala Gln

225 230 235 240

Lys Gly Pro Lys Asp Leu Lys Arg Leu Leu Pro Ser Thr Gln Pro Trp
245 250 255

Thr Cys Thr Ser Gln Arg Ala Val Ala Trp Thr Ser Leu Leu Ser Trp
260 265 270

Gly Ala Thr Thr Pro Arg Pro Ser Leu Trp Gln Ser Ala Ser Leu Pro
275 280 285

Arg Arg Arg Phe Cys Thr Ser Leu Ala Gly Arg Arg Lys Met Val Pro
290 295 300

Pro Pro Arg Pro Ser Cys Thr Thr Leu Met Arg Ala Cys Met Gly Ser
305 310 315 320

Ser Thr Gln Ser Cys Leu Thr Thr Ser Ala Leu Pro Pro Ser Cys Arg
325 330 335

Ser Leu Arg Thr Thr His Pro Ala Thr Cys Leu Ser Pro Phe Ile Ser
340 345 350

Leu Pro Cys Ser Ala Leu Leu
355

<210> 26
<211> 177
<212> PRT
<213> Human

<400> 26

Thr Gly Thr Gly Thr Thr Gly Cys Ala Thr Ala Cys Thr Thr Thr Cys
1 5 10 15

Thr Ala Ala Gly Gly Cys Gly Gly Cys Gly Gly Cys Thr Gly Cys Ala
20 25 30

Gly Cys Ala Gly Cys Gly Gly Cys Thr Cys Cys Ala Thr Cys Cys Ala
35 40 45

Gly Cys Cys Cys Gly Thr Cys Ala Gly Cys Thr Cys Cys Thr Cys Cys
50 55 60

Thr Gly Cys Ala Ala Gly Gly Cys Ala Thr Gly Gly Cys Thr Gly Gly
65 70 75 80

Cys Thr Ala Cys Cys Thr Gly Ala Gly Thr Gly Ala Ala Thr Cys Gly
85 90 95

Gly Ala Cys Thr Thr Thr Gly Thr Gly Ala Thr Gly Gly Thr Gly Gly
100 105 110

Ala Gly Gly Ala Gly Gly Gly Cys Thr Thr Cys Ala Gly Thr Ala Cys
115 120 125

Cys Cys Gly Ala Gly Ala Cys Cys Thr Gly Cys Thr Gly Ala Ala Gly
130 135 140

Gly Ala Ala Cys Thr Cys Ala Cys Thr Cys Thr Gly Gly Gly Gly Gly
145 150 155 160

Cys Cys Thr Cys Ala Cys Ala Gly Gly Cys Cys Ala Cys Cys Ala Cys
165 170 175

Gly

<210> 27
<211> 32
<212> DNA
<213> Human

<400> 27
aaactgccat ctaacatctg ctgaatggac tt 32

<210> 28
<211> 174
<212> DNA
<213> Human

<400> 28
gacgaggtag ctgccttctt cgtggctgac ctgggtgcca tagtgaggaa gcacttttgc 60

tttctgaagt gcctgccacg agtccggccc ttttatgctg tcaagtgcaa cagcagccca 120

ggtgtgctga aggttctggc ccagctgggg ctgggcttta gctgtgccaa caag 174

<210> 29
<211> 174
<212> DNA
<213> Human

<400> 29
gcagagatgg agttggtcca gcatattgga atccctgcca gtaagatcat ctgcgccaac 60

ccctgtaagc aaattgcaca gatcaaatat gctgccaagc atgggatcca gctgctgagc 120

tttgacaatg agatggagct ggcaaaggtg gtaaagagcc accccagtgc caag 174

<210> 30
<211> 135
<212> DNA
<213> Human

<400> 30
gatggttctg tgcattgcta ccgatgactc ccactccctg agctgcctga gcctaaagtt 60

tggagtgtca ctgaaatcct gcagacacct gcttgaaaat gcgaagaagc accatgtgga 120

ggtggtgggt gtgag 135

<210> 31
<211> 166
<212> DNA
<213> Human

<400> 31
ttttcacatt ggcagtggct gtcctgaccc tcaggcctat gctcagtcca tcgcagacgc 60

ccggctcgtg tttgaaatgg gcaccgagct gggtcacaag atgcacgttc tggaccttgg 120

tggtggcttc cctggcacag aaggggccaa agtgagattt gaagag 166

<210> 32
<211> 163
<212> DNA
<213> Human

<400> 32
attgcttccg tgatcaactc agccttggac ctgtacttcc cagagggctg tggcgtggac 60

atctttgctg agctggggcg ctactacgtg acctcggcct tcactgtggc agtcagcatc 120

attgccaaga aggaggttct gctagaccag cctggcaggg agg 163

<210> 33
<211> 113
<212> DNA
<213> Human

<400> 33
aggaaaatgg ttccacctcc aagaccatcg tgtaccacct tgatgagggc gtgtatggga 60

tcttcaactc agtcctgttt gacaacatct gccctacccc catcctgcag aag 113

<210> 34
<211> 70
<212> DNA
<213> Human

<400> 34
tctaagaacc actcaccctg ctacatgtct ctagagtcca ttcatttcat tgccgtgtag 60

cgctcttttg 70

<210> 35
<211> 215
<212> DNA
<213> Human

<400> 35
aaaccatcca cggagcagcc cctgtacagc agcagcctgt ggggcccggc ggttgatggc 60

tgtgattgcg tggctgaggg cctgtggctg ccgcaactac acgtagggga ctggctggtc 120

tttgacaaca tgggcgccta cactgtgggc atgggttccc ccttttgggg gacccaggcc 180

tgccacatca cctatgccat gtcccgggtg gcctg 215

<210> 36
<211> 583
<212> DNA
<213> Human

<400> 36
ggaagcgctg cgaaggcagc tgatggctgc agaacaggag gatgacgtgg agggtgtgtg 60

caagcctctg tcctgcggct gggagatcac agacaccctg tgcgtgggcc ctgtcttcac 120

cccagcgagc atcatgtgag tgggcctcgt tccccccgga gaatcccagc ggggcctcag 180

agatgcatct gggagaggtg gggaagatgg caggcaaggg tacccttggc caggactctg 240

gtgcccaccc tgccaccccc gcgctccacc tgcagtgttt ctgccctgta aataggacca 300

gtcttacact cgctgtagtt caagtatgca acataaatcc tgttccttcc agctgtgtct 360

gcctcctctg cagtgcaagg ggcctggtca gccaggtgtg gggtgttct tggggtctcc 420

tttggtctcc ttcccacctt tgtaaatata atgcaaataa ataaatattt aggtttttaa 480

aaactgcagc ggaatctggc agtttgattc acaaagcagc ctgggctagg cctggggcag 540

gatttcccca tcactcactg atgagcccac accctctgct tta 583